(19)

Europäisches Patentamt
European Patent Office
Office européen des brevets

(11)     EP 3 432 785 B1

(12)     **EUROPEAN PATENT SPECIFICATION**

| | |
|---|---|
| (45) Date of publication and mention of the grant of the patent: **10.03.2021 Bulletin 2021/10** | (51) Int Cl.: *C12Q 1/6806* $^{(2018.01)}$   *C12Q 1/6844* $^{(2018.01)}$ *G01N 21/64* $^{(2006.01)}$   *G01N 33/53* $^{(2006.01)}$ |
| (21) Application number: **17771315.3** | (86) International application number: **PCT/US2017/024328** |
| (22) Date of filing: **27.03.2017** | (87) International publication number: **WO 2017/165887 (28.09.2017 Gazette 2017/39)** |

(54) **POLARIZATION-BASED FLUORESCENT NUCLEIC ACID DETECTION**

POLARISATIONSBASIERTER FLUORESZIERENDER NUKLEINSÄURENACHWEIS

DÉTECTION D'ACIDE NUCLÉIQUE FLUORESCENT BASÉE SUR LA POLARISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2016 US 201662313427 P**

(43) Date of publication of application:
**30.01.2019 Bulletin 2019/05**

(73) Proprietor: **The General Hospital Corporation Boston, MA 02114 (US)**

(72) Inventors:
• **WEISSLEDER, Ralph**
  **Peabody, Massachusetts 01960 (US)**
• **LEE, Hakho**
  **Acton, Massachusetts 01720 (US)**
• **PARK, Kisoo**
  **Cambridge, Massachusetts 02139 (US)**
• **HUANG, Chen-Han**
  **Cambridge, Massachusetts 02129 (US)**

(74) Representative: **Fish & Richardson P.C. Highlight Business Towers Mies-van-der-Rohe-Straße 8 80807 München (DE)**

(56) References cited:
| | |
|---|---|
| WO-A1-2014/200767 | US-A1- 2002 055 091 |
| US-A1- 2011 117 025 | US-A1- 2011 236 960 |
| US-A1- 2012 003 631 | US-A1- 2014 046 150 |
| US-B2- 7 141 378 | |

• KI SOO PARK ET AL: "Target DNA induced switches of DNA polymerase activity", CHEMICAL COMMUNICATIONS, vol. 51, no. 49, 1 January 2015 (2015-01-01), pages 9942-9945, XP055530807, ISSN: 1359-7345, DOI: 10.1039/C5CC02060C
• PARK ET AL.: 'Fluorescence Polarization Based Nucleic Acid Testing for Rapid and Cost-Effective Diagnosis of Infectious Disease' CHEMISTRY: A EUROPEAN JOURNAL vol. 21, 09 November 2015, pages 16359 - 16363, XP055423541
• PARK ET AL.: 'Rapid Identification of Health Care-Associated Infections with an Integrated Fluorescence Anisotropy System' SCIENCE ADVANCES vol. 2, 01 May 2016, page E1600300, XP055423542

**Description**

**TECHNICAL FIELD**

This disclosure relates to polarization-based fluorescent nucleic acid detection.

**BACKGROUND**

Biological samples are often examined for the presence and prevalence of particular sequences of nucleic acids (e.g., ribonucleic acid molecules having a particular sequence of nucleic acids). In some cases, the presence and prevalence of particular sequences of nucleic acids can provide insight into a particular biological or pathogenic process, a progression of a particular disease, or some other biological condition of a subject. PARK ET AL, CHEMISTRY: A EUROPEAN JOURNAL, vol. 21, 9 November 2015, pages 16359-16363, relates to fluorescence polarization based nucleic acid testing for rapid and cost-effective diagnosis of infectious disease.

**SUMMARY**

The invention is set out in the appended set of claims. In an aspect, a method of detecting a target nucleic acid molecule in a sample includes incubating a sample with a reaction solution. The reaction solution includes a plurality of DNA polymerase molecules, a plurality of detector nucleic acid molecules, and a plurality of reporter molecules. Each detector nucleic acid molecule includes a first nucleic acid sequence that is complementary to a second nucleic acid sequence of a target nucleic acid molecule, and an aptamer that specifically binds to the DNA polymerase molecules. Each reporter molecule includes a third nucleic acid sequence, a primer molecule bound to the third nucleic acid sequence of the reporter molecule, and a fluorophore bound to the third nucleic acid sequence of the reporter molecule. Each detector nucleic acid molecule of the plurality of detector nucleic acid molecules is configured to hybridize with one or more corresponding target nucleic acid molecules. Each hybridized detector nucleic acid molecule is configured to bind to one or more of the DNA polymerase molecules such that the DNA polymerase molecules are inactivated. The method also includes directing excitation light to the sample to induce fluorescence by the fluorophores, measuring a level of anisotropy of the fluorescence, and determining a presence or an absence of the target nucleic acid molecule in the sample based on the level of anisotropy of the fluorescence.

Implementations of this aspect can include one or more of the following features.

In some implementations, for each reporter molecule of the plurality of reporter molecules, the plurality of DNA polymerase molecules can be configured to cleave the fluorophore from the reporter molecule. Inactivating a DNA polymerase molecule can inhibit cleaving of the fluorophore from the reporter molecule.

In some implementations, directing excitation light to the sample can include directing polarized light to the sample.

In some implementations, measuring the level of anisotropy of the fluorescence can include detecting fluorescence having a first polarization, detecting fluorescence having a second polarization that is different than the first polarization, and determining the level of anisotropy of the fluorescence based on the detected fluorescence having the first polarization and the detected fluorescence having the second polarization.

In some implementations, determining the presence or the absence of the target nucleic acid molecule in the sample can include determining that the measured level of anisotropy is greater than or equal to a threshold value, and responsive to determining that the measured level of anisotropy is greater than or equal to the threshold value, determining that the target nucleic acid molecule is present in the sample.

In some implementations, determining the presence or the absence of the target nucleic acid molecule in the sample can include determining that the measured level of anisotropy is less than a threshold value, and responsive to determining that the measured level of anisotropy is less than the threshold value, determining that the target nucleic acid molecule is absent in the sample.

In some implementations, the target nucleic acid molecule can be a ribonucleic acid (RNA) molecule.

In some implementations, the method can further include determining a presence of bacteria in the sample based on the measured level of anisotropy.

In some implementations, the method can further include distinguishing between two or more types of bacteria in the sample based on the measured level of anisotropy.

In some implementations, the method can further include detecting an indication of cancer based on the measured level of anisotropy.

In some implementations, the method can further include incubating the sample with a second reaction solution. The reaction solution can include a plurality of second DNA polymerase molecules, a plurality of second detector nucleic acid molecules, and a plurality of second reporter molecules. Each second detector nucleic acid molecule can include a fourth nucleic acid sequence that is complementary to a fifth nucleic acid sequence of a second target nucleic acid molecule, and a second aptamer that specifically binds to the second DNA polymerase molecules. Each second reporter molecule can include a sixth nucleic acid sequence, a second primer molecule bound to the sixth nucleic acid sequence of the second reporter molecule, and a second fluorophore bound to the sixth nucleic acid sequence of the second reporter molecule.

Each second detector nucleic acid molecule of the plurality of second detector nucleic acid molecules can be configured to hybridize with one or more corresponding second target nucleic acid molecules. Further, each hybridized second detector nucleic acid molecule can be configured to bind to one or more of the second DNA polymerase molecules such that the second DNA polymerase molecules are inactivated.

Second excitation light can be directed to the sample to induce second fluorescence by the second fluorophores. A level of anisotropy of the second fluorescence can be measured, and a presence or an absence of the second target nucleic acid molecule in the sample can be determined based on the level of anisotropy of the second fluorescence.

In another aspect, a system for detecting a target nucleic acid molecule in a sample includes one or more computer processors, and one or more measurement assemblies communicatively coupled to the one or more computer processors. Each measurement assembly includes a detection region configured to accept a sample, a light assembly configured and controlled by the one or more computer processors to direct excitation light into the sample in the detection region, a first photodetector assembly, and a second photodetector assembly. The first and second photodetector assemblies are configured and controlled by the one or more computer processors to measure a level of anisotropy of fluorescence emitted from the sample in the detection region. The one or more computer processors are configured to determine a presence or an absence of the target nucleic acid molecule in the sample based on the level of anisotropy of the fluorescence.

Implementations of this aspect can include one or more of the following features.

In some implementations, the light assembly can include a light source, and a first polarizer disposed between the light source and the detection region.

In some implementations, the first photodetector assembly can include a first photodiode, and a second polarizer disposed between the first photodiode and the detection region.

In some implementations, the second photodetector assembly can include a second photodiode, and a third polarizer disposed between the second photodiode and the detection region. A polarization of the second polarizer can be different than a polarization of the third polarizer.

In some implementations, the one or more computer processors can be enclosed in a housing. The one or more measurement assemblies can be reversibly attachable to the housing via a communications interface.

In some implementations, the system can include a plurality of measurement assemblies.

In some implementations, the system can further include a heating assembly. The heating assembly can be configured, during operation of the system, to apply heat to the detection region of each of the one or more measurement assemblies.

In some implementations, the system can further include a wireless transceiver configured to transmit data and/or receive data from a computing device external to the system.

In some implementations, the computer device can be a smartphone or a tablet computer.

In some implementations, the light assembly can be configured and controlled by the one or more computer processors to direct excitation light having a first wavelength into the sample in the detection region, and direct excitation light having a second wavelength into the sample in the detection region, wherein the first wavelength is different than the second wavelength.

In some implementations, the light assembly can be configured and controlled by the one or more computer processors to alternatingly direct excitation light having the first wavelength into the sample in the detection region and direct excitation light having the second wavelength into the sample in the detection region over a period of time.\

In another aspect, a sample preparation device includes a first compartment configured to enclose a sample, a second compartment configured to enclose a reaction solution, a partition disposed between the first compartment and the second compartment, and a plunger configured to puncture the partition in response to an applied force, such that the reaction solution enclosed within the second compartment mixes with the sample in the first compartment.

Implementations of this aspect can include one or more of the following features.

In some implementations, the plunger can include a shaft that extends through at least a portion of the second compartment along a longitudinal axis of the sample preparation device, and a flange mechanically coupled to the shaft. The flange can be external to the second compartment.

In some implementations, the plunger can be configured to puncture the partition using the shaft when the force is applied to the flange.

In another aspect, a kit includes an implementation of a system described herein and an implementation of a sample preparation device described herein.

In another aspect, a method includes providing a sample containing a polymerase molecule, a receptor molecule containing a fluorophore, and a detector molecule configured to, in the presence of a target nucleic acid, inhibit the polymerase molecule from cleaving the fluorophore. The method also includes detecting a level of fluorescence anisotropy from the sample to determine the absence or presence of the target nucleic acid in the sample.

The details of one or more embodiments are set forth in the accompanying drawings and the description below. Other features and advantages will be apparent from the description and drawings, and from the claims.

## BRIEF DESCRIPTION OF THE DRAWINGS

FIG. 1A is a diagram of an exemplary process for detecting target sequences of nucleic acids.

FIG. 1B is a diagram of an exemplary detector nucleic acid molecule and an exemplary reporter molecule.

FIG. 2 is a diagram of an exemplary instrument for measuring the fluorescent anisotropy of a sample.

FIG. 3 is a diagram of an exemplary measurement assembly.

FIGS. 4A-4C are photographs of an exemplary modular instrument for measuring the fluorescent anisotropy of a sample.

FIGS. 5A and 5B are diagrams of an exemplary integrated instrument for measuring the fluorescent anisotropy of a sample.

FIG. 6A is a diagram of an exemplary sample preparation device.

FIG. 6B is a diagram of an exemplary usage of the sample preparation device shown in FIG. 6A.

FIGS. 7A-7D are diagrams of an exemplary device to isolate or extract nucleic acid molecules.

FIGS. 8A-8C are diagrams of an exemplary control application associated with an instrument.

FIG. 9 is a flow chart diagram of an exemplary process of detecting a target nucleic acid molecule in a sample.

FIG. 10 is a diagram of an exemplary computer system.

FIGS. 11A-C are circuit diagrams of an exemplary system or detecting target sequences of nucleic acids.

FIG. 12 is a plot showing a performance comparison between an exemplary system and a commercial benchtop plate reader.

FIG. 13 is a plot showing measurements obtained from five different HAI pathogens using an exemplary system.

FIG. 14 is a bar graph showing a detection sensitivity of an exemplary system for samples with different bacterial concentrations.

FIG. 15 is a schematic illustration of uracil DNA glycosylase (UDG)-mediated control on carryover contamination.

FIG. 16 is a bar graph showing an exemplary effect of dUTP on a system assay.

FIG. 17 is a bar graph showing the results of an exemplary contamination control technique.

FIG. 18 is a bar graph showing a performance comparison between an exemplary system and conventional bench-top equipment.

FIGS. 19 and 20 are bar graphs showing measurements obtained using exemplary fresh and stored reagents using an exemplary system.

FIG. 21 is a diagram showing an exemplary detector nucleic acid molecule.

FIGS. 22 and 23 are graphs showing the results of a specificity test conducted using an exemplary system.

FIG. 24 is a photograph showing electrophoretic band-shift analyses conducted to confirm the results of the specify test shown in FIGS. 22 and 23.

FIG. 25A and 25B are graphs showing the result of detecting drug-resistance and virulence factors using an exemplary system.

FIG. 26 shows an exemplary process for assaying clinical samples.

FIGS. 27 and 28 are graphs showing measurements obtained from clinical samples using an exemplary system.

FIG. 29 is a show a comparison between results obtained using an exemplary system and results obtained through qPCR.

FIG. 30 shows exemplary cells and their mutation status.

FIG. 31 shows results of a process for improving a detection specificity of an exemplary system.

FIG. 32 shows a profiling of somatic mutations in exemplary cells and their secreting exosomes.

## DETAILED DESCRIPTION

Systems and techniques for detecting sequences of nucleic acids are described herein. One or more of the implementations described herein can be used to identify the presence or the absence of nucleic acid molecules having particular sequences of nucleic acids (e.g., sequences indicative of particular biological conditions of a subject) using a fluorescent-based technique. The resulting information can be used to provide more effective care to the subject (e.g., by enabling caretakers to make diagnoses and/or administer treatment in a more informed manner).

In some cases, the implementations described herein can be used to detect biological conditions, such as healthcare-associated infections (HAIs) by detecting the presence of HAI pathogens (e.g., particular bacterial species). In some case, the implementations described herein can be used to detect the onset and progression of cancers.

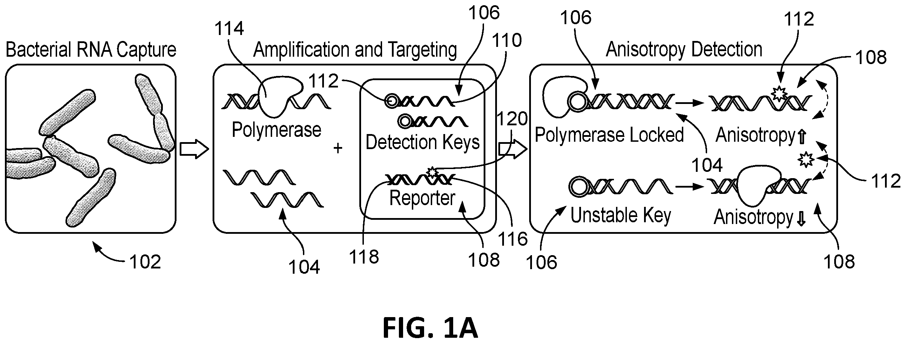

FIG. 1A is a schematic that illustrates an example of a process for detecting whether a biological particle within a sample contains a target sequence of nucleic acids (e.g., a sequence of nucleic acid indicative of a particular biological condition). As shown in FIG. 1A, a sample 102 containing a biological particle (e.g., a sample containing bacteria) is obtained. While the biological particle depicted in the example of FIG. 1A includes bacteria, the sample 102 may contain, e.g., one or more various microbes, cells, viruses or pathogens. In the present example, the bacteria in the sample are modified (e.g., lysed) to extract nucleic acids 104 contained within the bacteria. The nucleic acid molecules 104 extracted from the bacteria may include a target nucleic acid sequence (e.g., regions in 16S rRNA or mRNA) and are amplified (e.g., via asymmetric reverse-transcription (RT) PCR).

After amplification, a reaction solution is combined with the sample. The reaction solution includes two reagents: (i) detector nucleic acid molecules 106, and (ii) reporter molecules 108. In addition, DNA polymerase molecules 114 are combined with the reaction solution-sample mixture.

The detector nucleic acid molecule 106 includes a first nucleic acid sequence 110 that is complementary to the target nucleic acid sequence. The detector nucleic acid molecule 106 further includes an aptamer 112 that specifically binds to DNA polymerase molecules 114.

In practice, the first nucleic acid sequence 110 can differ, depending on the target nucleic acid molecule 104 being detected. In some cases, the first nucleic acid sequence 110 can be designed such that the detector nucleic acid molecule 106 selectively binds to a nucleic acid sequence indicative of a particular biological condition (e.g., HAI or cancer). In some cases, the first nucleic acid sequence 110 can be designed such that the detector nucleic acid molecule 106 selectively binds to a nucleic acid sequence indicative of a particular type of organism (e.g., a particular type of bacteria).

Each reporter molecule 108 includes a second nucleic acid sequence 116, a primer molecule 118 bound to the second nucleic acid sequence 116, and a fluorophore 120 bound to the nucleic acid sequence.

In a first case, the nucleic acid molecules 104 do not include the target nucleic acid sequence. In the absence of the target nucleic acid molecules 104, the DNA polymerase 114 then binds to the reporter molecules 108, and synthesizes DNA molecules from the reporter molecules 108. During the synthesis reaction, the DNA polymerase cleaves the fluorophores from the reporter molecules 108. Light (e.g., polarized light) having a first wavelength then is directed to the mixture. When the fluorophores are excited by the incident light, the fluorophores emit light at a second different wavelength, which can be detected. Due to the relatively high diffusional motion of the unbound fluorophores 112, the light emitted by the mixture exhibits a relatively low fluorescent anisotropy (e.g., ratio of emitted light polarized along a first direction relative to emitted light polarized along a second different direction).

In a second case, the nucleic acid molecules 104 include the target nucleic acid sequence. In this case, the detector nucleic acid molecules 106 hybridize with the target nucleic acid molecules 104, stabilizing the detector nucleic acid molecules 106. The resulting hybridized detector nucleic acid molecule 106 binds to the DNA polymerase 114, and deactivates the enzymatic activity of the DNA polymerase 114. As a result, DNA synthesis is inhibited, and the fluorophores 120 of the reporter molecules 108 are not cleaved from the reporter molecules 108.

Light (e.g., polarized light) having a first wavelength then is directed to the mixture. The excited fluorophores emit light at a second different wavelength that can be detected. In contrast to the first case, the fluorescent anisotropy of the detected light may be relatively high due, e.g., to the relatively low diffusivity of the still-bound fluorophores.

Accordingly, the presence or the absence of the target nucleic acid molecules can be ascertained by incubating a sample with the reaction solution, inducing fluorescence in the sample, and measuring the anisotropy of the resulting fluorescence

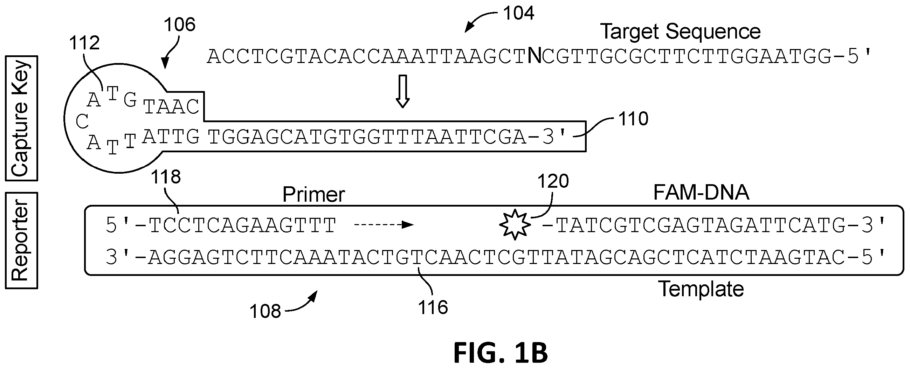

An example detector nucleic acid molecule 106 and example reporter molecule 108 is shown in FIG. 1B. As shown in FIG. 1B, the detector nucleic acid molecule 106 includes a first nucleic acid sequence 110 that is complementary to a target nucleic acid sequence. The detector nucleic acid molecule 106 includes an aptamer 112 that specifically binds to DNA polymerase molecules 114. Although example nucleic acid sequences are shown in FIG. 1B, is it understood that these are merely examples. In practice, other nucleic acid sequences also can be used (e.g., to probe for different target nucleic acid sequences).

Further, as shown in FIG. 1B, each reporter molecule 108 includes a second nucleic acid sequence 116, a primer molecule 118 bound to the second nucleic acid sequence 116, and a fluorophore 120 bound to the second nucleic acid sequence 116.

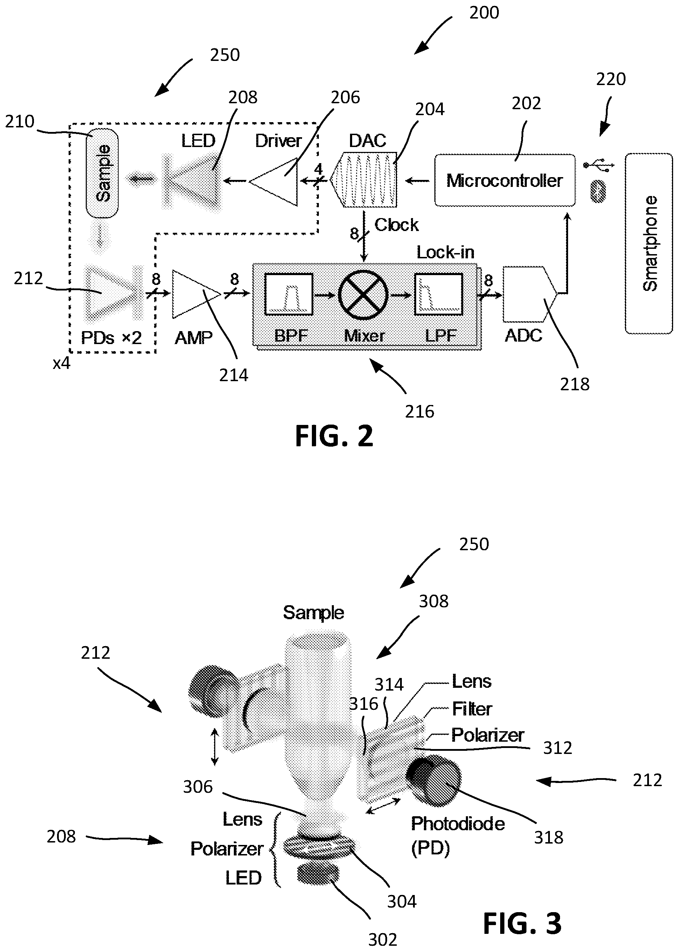

FIG. 2 is a schematic diagram of an exemplary instrument 200 for measuring the fluorescent anisotropy of a sample. In some cases, the system 200 is referred to as a polarization anisotropy diagnostics (PAD) device.

The instrument 200 includes a microcontroller 202 that generates a digital carrier signal (e.g., a periodic time-varying digital signal). The digital carrier signal is converted into an analog carrier signal using a digital-to-analog converter (DAC) 204. The analog carrier signal is transmitted to a measurement assembly 250.

The measurement assembly 250 includes a driver 206 that receives the analog carrier signal from the DAC 204, amplifies the analog carrier signal, and supplies the amplified carrier signal to a lighting assembly 208 (e.g., an assembly including one or more light emitting diodes (LEDs)) 208. The lighting assembly 208 illuminates in response to the amplified carrier signal, generating excitation light that varies in time according to the carrier signal. The excitation light is directed to a sample 210 (e.g., a sample containing a biological sample from a subject, incubated with a reaction solution with detector nucleic acid molecules and reporter molecules). The intensity of the induced fluorescent is measured using photodetector assemblies 212 (e.g., assemblies including one or more photodetectors (PDs)).

The output of the photodetector assemblies 212 are amplified by corresponding amplifiers 214, and are processed using signal processing module 216. The signal processing module 216 filters the output of the amplifiers 214 (e.g., using a band pass having a center frequency matching that of the carrier signal), and mixes the filtered output with the analog carrier signal from the DAC 204. The signal processing module 216 performs a "lock in" procedure (described further below) to increase the signal-to-noise ratio (SNR) of the output. Although the signal processing module 216 is shown as a separate component in FIG. 1A, this need not be the case. For example, in some implementations, the signal processing module 216 can be implemented as a part of a photodetector assembly 212. In some implementations, the signal processing module 216 can be implemented as a part of the microcontroller 202.

The output of the processing module is converted to digital signals using analog-to-digital converters 218, and is transmitted to the microcontroller 202 for interpretation and/or further processing.

The instrument 200 also includes a communications interface 220 that enables the instrument to communicate with other computing devices. As an example, the communications interface 220 can include a connection port (e.g., a universal serial bus (USB) port) and/or a wireless transceiver (e.g., a Bluetooth or Wi-Fi transceiver) to facilitate communications with an external computing device (e.g., a smartphone, a personal computer, a tablet computer, among other computing devices.). In some cases, the external computing device can be used to initialize and configure the instrument, view data from the instrument, process data from the instrument, and/or store data for future retrieval.

An example of a measurement assembly 250 is shown in greater detail in FIG. 3. As shown in FIG. 3, a lighting assembly includes an LED 302, a linear polarizer 304, and a lens 306. In response to a drive current (e.g., supplied by a driver 206), the LED 302 generates excitation light. The excitation light is linearly polarized by the linear polarizer 304, and focused into a detection region 308 using the lens 306. When a sample 310 is inserted into the detection region 308, the linearly polarized excitation light excites the fluorophores in the sample, causing the fluorophores to fluoresce.

The fluorescence emitted from the sample is measured by the photodetector assemblies 212. In the present example, two separate photodetector assemblies are provided, on opposite sides of the sample. Each photodetector assembly 212 includes a linear polarizer 312, a filter 314, a lens 316, and a photodiode 318. The lens 316 focuses fluorescence from the sample region 308 through the filter 314 (to attenuate and/or remove wavelengths of light not associated with the fluorescence) and through the linear polarizer 312 onto the photodiode 318. The polarization of each linear polarizer 312 is different (e.g., the polarization of one linear polarizer 312 can be orthogonal to the polarization of another linear polarize 312). Accordingly, each of the photodiodes 318 measures a different directional component of the intensity of the fluorescence in the sample region 308.

As shown in FIG. 3, the multiple photodetector assemblies 212 can be used to measure the anisotropy of the fluorescence. For example, a first photodiode 318 from a first photodetector assembly 212 can be used to measure a parallel polarization component of the intensity of fluorescence ($I_x$) relative to the excitation light polarization, and a second photodiode 318 from a second photodetector assembly 212 can be used to measure a perpendicular polarization component of the intensity of fluorescence ($I_y$) relative to the excitation light polarization. The anisotropy of the fluorescence, $r$, can be calculated as:

$$r = \left(I_x - I_y\right)\left(I_x + 2I_y\right)^{-1}.$$

As described herein, a "lock-in" measurement techniques can be used to increase the SNR of the measurements. For instance, exploiting the fact that $r$ is intensity-independent, the measured intensity of the excitation light can be modulated at the carrier frequency (e.g., as generated by the microcontroller 202 and converted using the DAC 204). The emission signals can then be then frequency-locked through a homodyne signal path.

As described herein, the presence or the absence of the target nucleic acid molecules can be ascertained by measuring the anisotropy of the fluorescence induced in the sample. For example, a relatively high level of anisotropy (e.g., greater than or equal to a particular threshold value) can indicate the presence of the target nucleic acid molecules, or a relatively low level of anisotropy (e.g., less than a particular threshold value) can indicate the absence of the target nucleic acid molecules. Threshold values can be empirically determined (e.g., by conducting experiments with positive samples known to include the target nucleic acid molecules and negative samples known not to have the target nucleic acid molecules, and measuring the resulting level of fluorescent anisotropy).

In some cases, a relative abundance of a target nucleic acid can be ascertained by measuring the anisotropy of the fluorescence induced in the sample. For example, increasing anisotropy levels can correspond to increasing abundances or amounts of the target nucleic acid. Further, decreasing anisotropy levels can correspond to decreasing abundances or amounts of the target nucleic acid. The relationship between observed level of anisotropy and abundance of target nucleic acids can be empirically determined (e.g., by conducting experiments with samples having known amounts of the target nucleic acid molecules, and measuring the resulting level of fluorescent anisotropy).

The instrument 200 for measuring the fluorescent anisotropy of a sample can include any number of measurements assemblies 250. In some implementations, the instrument 200 can include a single measurement assembly 250 to conduct a single measurement at a time. This can be beneficial, for example, in reducing the cost, complexity, and/or the size of the instrument 200. In some implementations, an instrument 200 can include multiple separate measurement assemblies 250 to conduct multiple measurements in parallel and/or sequentially. This can be beneficial, for example, as it enables multiple samples to be analyzed more quickly and/or more efficiently. As an example, as shown in FIG. 2, an instrument can include four measurement assemblies 250.

In some cases, an instrument 200 can be modular, such that measurement assemblies 250 can be reversibly attached and detached from the instrument 200. This can be useful, for example, as it enables users to service the instrument 200 more easily (e.g., by selectively replacing components). Further, this enables users to customize the instrument 200 according to their needs.

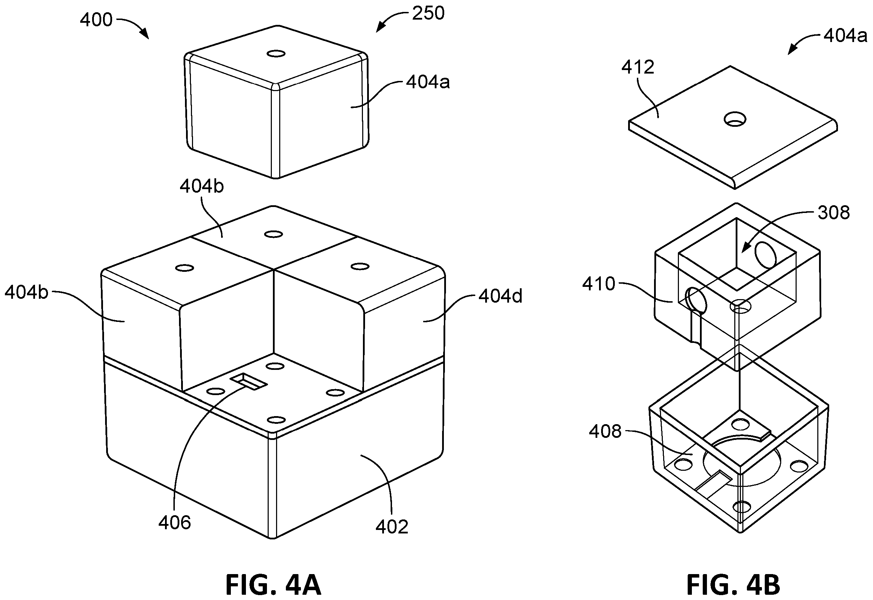

An exemplary modular instrument 400 is shown in FIG. 4A. The instrument 400 includes a base station 402, and modules 404a-d. The base station 402 contains components common to each of the modules 404a-d (e.g., a microcontroller 202, a DAC 204, amplifiers 214, signal processing modules 216, ADCs 218, and/or communications interfaces 220, as shown in FIG. 2). Each module 404a-d contains a measurement module 250. The modules 404a-d and the base station 402 can be communicatively coupled via one or more communications interfaces 406 (e.g., a port or connector).

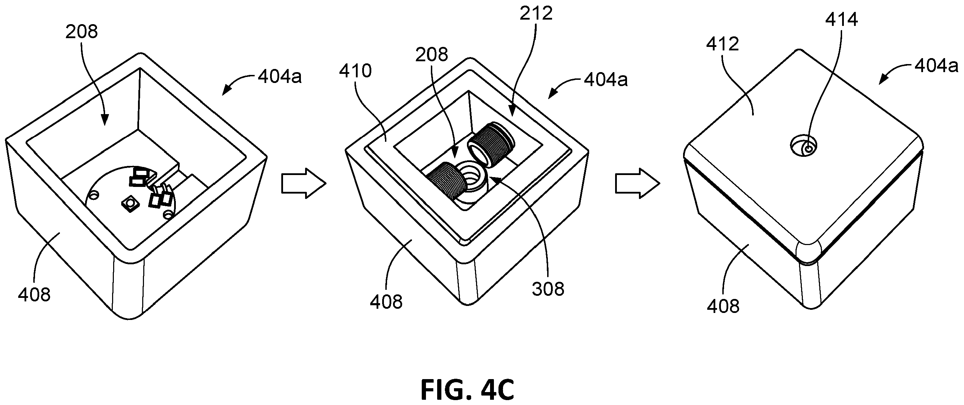

FIG. 4B shows an exploded view of the module 404a. The module 404a includes a housing 408, a support frame 410, and a lid 412. As shown in FIG. 4C, the housing 408 includes a lighting assembly 208 positioned along its bottom interior surface. The support frame 410 inserts into the housing 408, and secures the lighting assembly 208 and the photodetector assemblies about the detection region 308. The lid 412 defines an aperture 414, though which a sample (e.g., a vessel, such a test tube, containing the sample) can be inserted into the detection region 308.

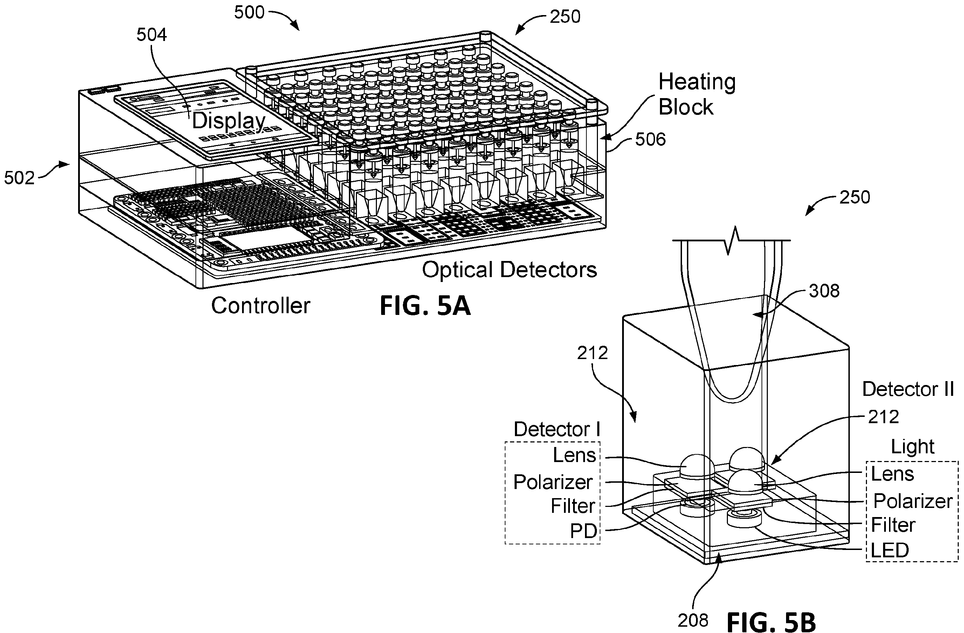

In some cases, an instrument can include several measurement assemblies in a single integrated system. For example, FIG. 5A shows an instrument 500 having 64 measurement assemblies 250, and an enclosure 502 (containing components common to each of the measurement assemblies 250, such as a microcontroller 202, a DAC 204, amplifiers 214, signal processing modules 216, ADCs 218, and/or communications interfaces 220, as shown in FIG. 2).

The instrument 500 also includes a display device 504 (e.g., a touch-sensitive display screen) for representing information to a user and/or for receiving inputs from a user (e.g., touch-based commands).

The instrument 500 also includes a heating assembly 506 (e.g., an electrically powered heating block) that heats and/or cools samples inserted into the measurement assemblies 250. This can be useful, for example, as it enables the instrument 500 to function as a thermo-cycler, which may facilitate the performance of PCR.

In some cases, the heating assembly 506 can include positive-temperature-coefficient (PTC) heating elements. PTC heating elements can be useful in simplifying feedback control of the heating assembly 506. For example, PTC heating elements initially have low electrical resistance to rapidly generate heat. As the temperature rises, the resistance increases to self-regulate the electrical current, preventing overheating.

A single measurement assembly 250 of the instrument 500 is shown in greater detail in FIG. 5B. As shown in FIG. 5B, each measurement assembly includes a lighting assembly 208, and two photodetector assemblies 212 positioned about a detection region 308. A sample (e.g., a vessel containing a sample) can be inserted into the detection region 308 for measurement.

In some cases, multiple sets of detector nucleic acid molecules, reporter molecules, and DNA polymerase molecules can be used in conjunction to detect multiple different target nucleic acid molecules.

For example, a first set can be used to detect first target nucleic acid molecules. The first set can include first detector nucleic acid molecules, first reporter molecules having first fluorophores, and first DNA polymerase molecules. The first detector nucleic acid molecules include first nucleic acid sequences that are complementary to a nucleic acid sequence of the first nucleic acid molecules. The first detector nucleic acid molecules also include first aptamers that specifically bind to the first DNA polymerase molecules (e.g., when the first detector nucleic acid molecules hybridize with the first target nucleic acid molecules).

In the presence of the first target nucleic acid molecules, the first DNA polymerase molecules are inactivated. In this case, when the first fluorophores are excited by incident light, the light emitted by the first fluorophores exhibit a relatively high fluorescent anisotropy. In the absence of the first target nucleic acid molecules, the first DNA polymerase molecules bind to the first reporter molecules, and synthesize DNA molecules from the first reporter molecules. In this case, when the first fluorophores are excited by incident light, the light emitted by the first fluorophores exhibit a relatively low fluorescent anisotropy. Thus, the presence or the absence of the first target nucleic acid molecules can be ascertained based on the fluorescent anisotropy associated with the first fluorophores. Further. a second set can be used to detect second target nucleic acid molecules. The second set can include second detector nucleic acid molecules, second reporter molecules having second fluorophores (e.g., having a different excitation wavelength and/or a different emission wavelength than the first fluorophores), and second DNA polymerase molecules. The second detector nucleic acid molecules include second nucleic acid sequences that are complementary to a nucleic acid sequence of the second nucleic acid molecules. The second detector nucleic acid molecules also include second aptamers that specifically bind to the second DNA polymerase molecules (e.g., when the second detector nucleic acid molecules hybridize with the second target nucleic acid molecules).

In the presence of the second target nucleic acid molecules, the second DNA polymerase molecules are inactivated. In this case, when the second fluorophores are excited by incident light, the light emitted by the second fluorophores exhibit a relatively high fluorescent anisotropy. In the absence of the second target nucleic acid molecules, the second DNA polymerase molecules bind to second first reporter molecules, and synthesize DNA molecules from the second reporter molecules. In this case, when the second fluorophores are excited by incident light, the light emitted by the first fluorophores exhibit a relatively low fluorescent anisotropy. Thus, the presence or the absence of the second target nucleic acid molecules can be ascertained based on the fluorescent anisotropy associated with the second fluorophores.

A sample can be incubated with multiple sets of detector nucleic acid molecules, reporter molecules, and DNA polymerase molecules concurrently. Subsequently, light having different wavelengths (e.g., corresponding to the excitation wavelengths of the different fluorophores) can be directed to the samples, and an intensity of light emitted by the samples can be measured according to multiple different wavelengths (e.g., corresponding to the emission wavelengths of the different fluorophores). Thus, the presence or the absence of different target nucleic acid molecules can be ascertained based on the fluorescent anisotropy associated with each fluorophore.

In some cases, an instrument can include a lighting assembly that produces excitation light according to different wavelengths, such that different types of fluorophores can be selectively excited and their fluorescent anisotropy observed. For example, a lighting assembly can have a first LED that emits light according to a first wavelength (corresponding to the excitation wavelength of a first fluorophore), and a second LED that emits light according to a different second wavelength (corresponding to the excitation wavelength of a second fluorophore), The instrument can selectively activate the first and second LEDs to probe the first and second fluorophores, respectively. In some cases, the instrument can cyclically activate the first and second LEDs in an alternating manner over a period of time to obtain multiple measurements for each type of fluorophore. In some cases, the instrument can also selectively activate the photodetector assemblies in sync with the activation of the first and second LEDs, such that measurements are obtained substantially only when the first or second LEDs are emitting light.

Although the use of two different sets of detector nucleic acid molecules, reporter molecules, and DNA polymerase molecules is described above, this is merely an illustrative example. In some cases, three of more sets of detector nucleic acid molecules, reporter molecules, and DNA polymerase molecules can be used to detect three or more different target nucleic acid molecules. Similarly, instruments can include light assemblies configured to produce excitation light according to any number of different wavelengths, such that different types of fluorophores can be selectively excited and their fluorescent anisotropy observed.

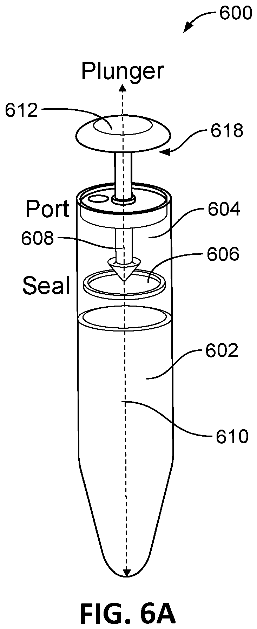

In some cases, a sample preparation device can be used to simplify the preparation of samples. An exemplary sample preparation device 600 is shown in FIG. 6A. The device 600 defines a first compartment 602 and a second compartment 604 separated by a partition 606. The partition 606 seals the compartment 602 from the second compartment 604, such that the contents of each cannot mix. In some cases, the partition 606 can be a plastic membrane or a sheet of foil (e.g., aluminum foil).

The device 600 also includes a plunger 618 configured to puncture the partition 606 in response to an applied force, such that the contents of the compartments 602 and 604 mix. The plunger 618 includes a shaft 608 that extends through the compartment 604 along a longitudinal axis 610 of the device 600, and a flange 612 external the compartment 604 and protruding from the device 600. A force can be applied to the flange 612 (e.g., by a user and/or a mechanical actuator or servo pressing on the flange 612), to shift the plunger 618 further into the device 600. When the plunger 618 is pressed sufficiently far into the device 600, the shaft 608 punctures the partition 606 (e.g., via a barbed tip), enabling the contents of the compartment 602 and 604 to mix.

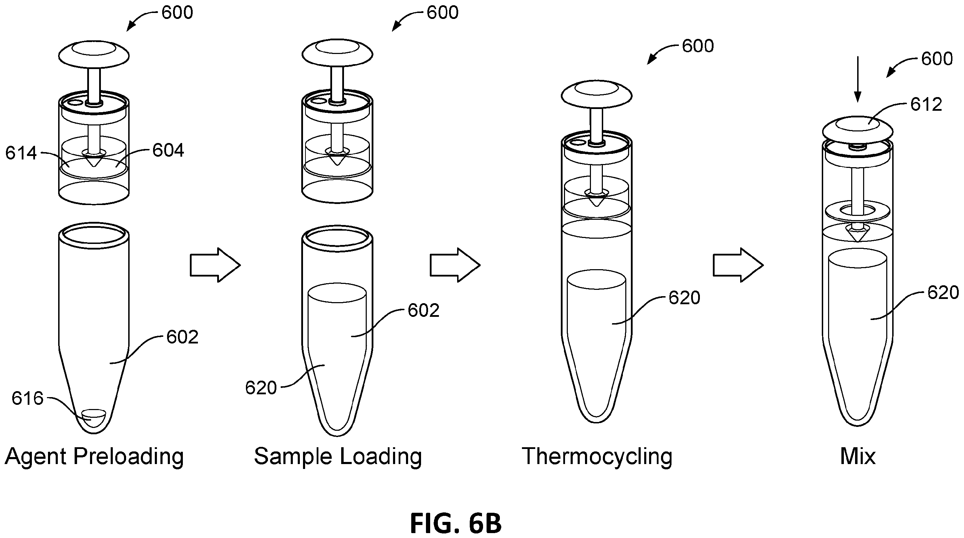

The device 600 enables samples to be prepared quickly and conveniently for use in the instruments described herein. For example, as shown in FIG. 6B, the device 600 can be pre-loaded with the reaction solution 614 in the compartment 604, and PCR reagents 616 in the compartment 602. PCR reagents 616 can include, for example, DNA polymerase, dNTP, primers, and buffers (e.g., when detecting DNA targets). PCR reagents 616 can also include reverse transcriptase (e.g., when detecting RNA targets).

A user can deposit a sample 620 into the compartment 602, such that it mixes with the PCR reagents. The user can place the device 600 into a thermocycler (e.g., the instrument 500 or a separate thermocycler) to amplify the target nucleic acid molecules in the sample 620 via PCR. After amplification is complete, the flange 612 can be pressed so that the partition is punctured and the reaction solution is released into the sample 620. Subsequently, the sample 620 can be incubated, and the fluorescent anisotropy can be measured to determine the presence or the absence of the target nucleic acid sequence (e.g., using one or more of the instruments described herein).

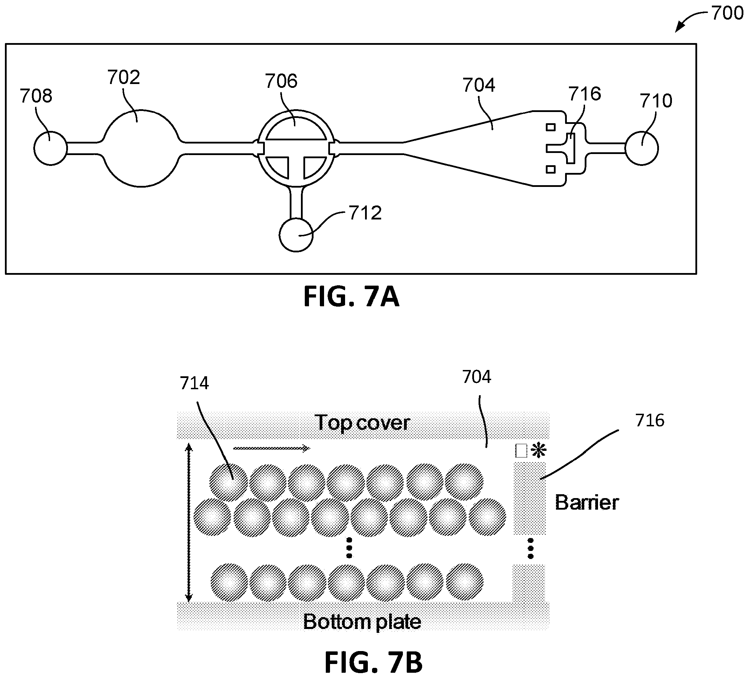

In some cases, a sample can be processed to isolate or extract nucleic acid molecules (e.g., RNA molecules) prior to being analyzed by a measurement instrument. As an example, FIG. 7A shows a cross sectional view of a nucleic acid extraction device 700. The device 700 includes a sample reservoir 702 and a capture chamber 704 in fluid communication via a valve 706 (e.g., a three-way valve). The device 700 also includes an inlet 708 for depositing fluid samples into the sample reservoir 702, an outlet 710 for retrieving fluid samples from the capture chamber 704, and a waste outlet 712 for removing waste material from the device 700.

The valve 706 can be adjusted to divert fluids between the inlet 708, the outlet 710, and/or the waste outlet 712. For example, in a first position, the valve 706 can allow for the flow of material between the inlet 708 and the outlet 710 (e.g., to carry samples from the inlet 708, through the sample reservoir 702 and the capture chamber 704, and out from the outlet 710). In a second position, the valve 706 can allow for the flow of material between the inlet 708 and the waste outlet 712 (e.g., to carry wash buffer or other materials) without contaminating the capture chamber 704. For instance, in some cases, magnetically labeled samples (e.g., exosomes captured on magnetic beads) can be immobilized in the sample reservoir 702 (e.g., using an external magnet), and the samples can be washed (e.g., using a wash buffer). During washing, the valve 706 can be set to the second position, such that the wash buffer does not enter the capture chamber 704, and is instead removed via the waste outlet 712.

A side view through a portion of the capture chamber 704 is shown in greater detail in FIG. 7B. The capture chamber 704 includes several positively-charged glass beads 714 packed within it. Under high salt conditions, negatively charged nucleic acid molecules bind to the surfaces of the glass beads. The nucleic acid molecules can be subsequently collected by reducing the salt concentration, and collecting the elutant from the outlet 710. As shown in FIG. 7B, the capture chamber 704 includes a weir-style barrier 716 to retain the beads 714 within the capture chamber 704 while allowing for fluidic flow.

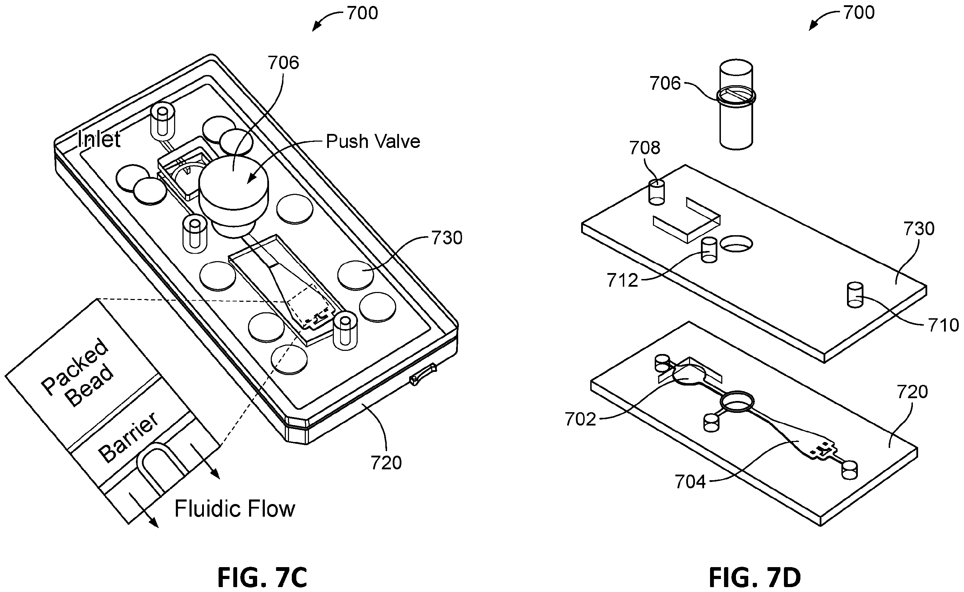

In some cases, the device 700 can be constructed by machining, etching, or molding features in a substrate (e.g., poly-methylmethacrylate (PMMA)), and sealing the features (e.g., using one or more covers or plates overlaid over the substrate). For example, an assembled view and an exploded view of the device 700 are shown in FIGS. 7C and 7D, respectively. As shown in FIGS. 7C and 7D, the device 700 can include a substrate 720, onto which the sample reservoir 702 and a capture chamber 704 are defined. The device 700 can also include a cover 730 defining the inlet 708, the outlet 710, and the waste outlet 712. The valve 706 is inserted into the cover 730 to regulate the flow of fluid between the sample reservoir 702 and a capture chamber 704.

As described herein, in some cases, an external computing device (e.g., a smartphone, a personal computer, a tablet computer, etc.) can be used to initialize and configure the instrument, view data from the instrument, process data from the instrument, and/or store data for future retrieval. In some cases, consumers can be provided with a control application for a computing device that enables the consumer to perform these tasks.

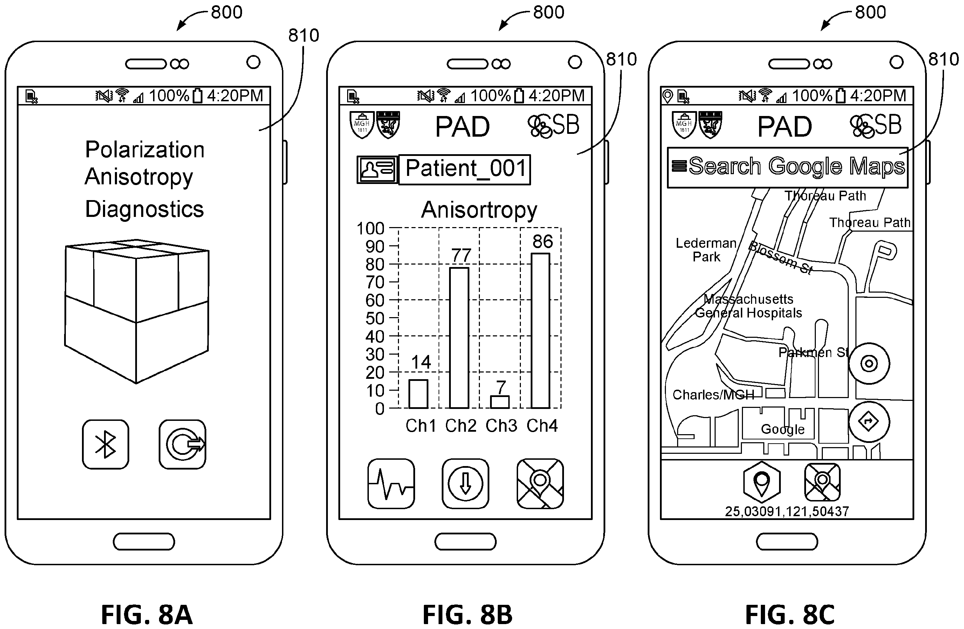

To illustrate, FIGS 8A-C show a smartphone 800 executing a control application associated with an instrument. The control application presents a graphical user interface (GUI) 810 to the user, presenting various options for initializing and configuring the instrument (FIG. 8A), viewing data from the instrument (FIG. 8B), and processing and/or storing data for future retrieval (FIG. 8C). As shown in FIG. 8C, in some cases, data can be combined with geographical information (e.g., the location where each measurement was made), and the data can be visually presented in the form of a graphical map. This can be useful, for example, if the user processes different samples from different geographical locations, and wishes to visually organize measurements according to their respective locations.

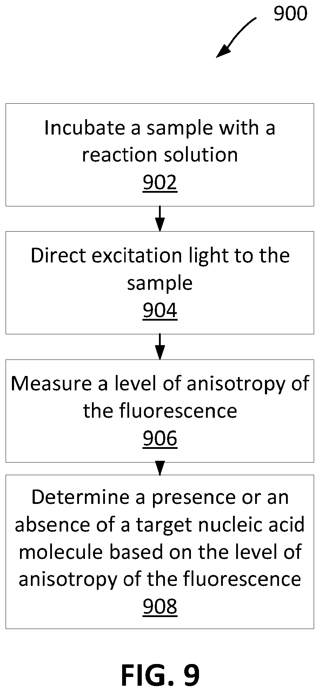

An example process 900 of detecting a target nucleic acid molecule in a sample is shown in FIG. 9.

A sample is incubated with a reaction solution (step 902).

Samples can include biological fluids, such as blood, cerebrospinal fluid (CSF), saliva, urine, sputum, puss, and/or food. Biological particles contained in the sample can include, e.g., microbes, cells (including any prokaryotic or eukaryotic cells), organelles, extracellular vesicles (e.g., exosomes), viruses, fungi, algae or other types of biological particles that occur either naturally or are introduced artificially into the sample. Examples of cells that can be used as a biological particle include, but are not limited to, circulating tumor cells, red blood cells, white blood cells, sperm, and eggs.

The reaction solution includes a plurality of DNA polymerase molecules, a plurality of detector nucleic acid molecules, and a plurality of reporter molecules.

Each detector nucleic acid molecule includes a first nucleic acid sequence that is complementary to a second nucleic acid sequence of a target nucleic acid molecule, and an aptamer that specifically binds to the DNA polymerase molecules. The target nucleic acid molecule can be a ribonucleic acid (RNA) molecule.

Each reporter molecule includes a third nucleic acid sequence, a primer molecule bound to the third nucleic acid sequence of the reporter molecule, and a fluorophore bound to the third nucleic acid sequence of the reporter molecule.

A variety of different fluorophores can be used. Example fluorophores include Alexa 405 and Cascade blue (having an excitation wavelength of 400 nm and an emission wavelength of 420 nm); Alexa 488 and FITC (having an excitation wavelength of 493 nm and an emission wavelength of 518 nm); Alexa 546, Alexa 555, Cy3, and TRITC (having an excitation wavelength of 550 nm and an emission wavelength of 568 nm); Alexa 594 and Texas red (having an excitation wavelength of 593 nm and an emission wavelength of 618 nm); Alexa 633 (having an excitation wavelength of 638 nm and an emission wavelength of 658 nm); Alexa 647 and Cy5 (having an excitation wavelength of 646 nm and an emission wavelength of 674 nm); Alexa 680 and Cy5.5 (having an excitation wavelength of 682 nm and an emission wavelength of 715 nm); and IRDye 800 (having an excitation wavelength of 770 nm and an emission wavelength of 794 nm). Other fluorophores also can be used, depending on the implementation.

A variety of different aptamers can be used. For instance, to deactivate Taq polymerase, aptamers having one of the following sequences can be used: CAAGACG ggCGggTGTggTAgg CGCCCGTG, ACTTGAT ggCGggTGTggTAgg CGCCATCT, ACTTGAT ggCGggTTTggTAgg CGCCGTCG, ACTGGAT ggCGggTTTggTAgg CGCCATCT, ACATGAT ggCGggTGTggTAgg CGTCGTCT, ACATGAT ggCGggTTTggTAgg CGCCGTCT, CAAGACG ggCGggTTTggTAgg CGCCCGAG, CAAGACG ggCGggTTTggGAgg CGCCCGAG, ACTTGAT ggCGggTTTggTAgg CGCCCTCT, CAAGACG ggCGggTTTggTAgg CGCCGTCT, ACTTGAT ggCGggTTTggTAgg CGCCCCAG, ACTTGAT ggTGggTTTgg-TAgg CGCCGTCT, ACAGACG ggCGggTTTggTAgg CGCCGACT, CCTGACG ggCGggTTTggTAgg CGCCGTCT, and ACAGACG ggCGggTTCggTAgg CGCCCTAG. Aptamers having other sequences also can be used, depending on the implementation. In some cases, aptamers can be generate through a systematic evolution of ligands by exponential enrichment (SELEX) process.

Each detector nucleic acid molecule of the plurality of detector nucleic acid molecules is configured to hybridize with one or more corresponding target nucleic acid molecules. Further, each hybridized detector nucleic acid molecule is configured to bind to one or more of the DNA polymerase molecules such that the DNA polymerase molecules are inactivated.

Excitation light is directed to the sample to induce fluorescence by the fluorophores (step 904). As an example, an instrument having a lighting assembly can be used to generate light (e.g., polarized light) having a first wavelength corresponding to the excitation wavelength of the fluorophores.

A level of anisotropy of the fluorescence is measured (step 906). As an example, an instrument having a lighting assembly can be used to detect light at a second different wavelength corresponding to the emission wavelength of the fluorophores. The anisotropy of the fluorescent can be determined by determining the ratio of emitted light polarized along a first direction relative to emitted light polarized along a second different direction. For instance, an instruction can detect fluorescence having a first polarization, and fluorescence having a second polarization that is different than the first polarization. The level of anisotropy of the fluorescence can be determined based on the detected fluorescence having the first polarization and the detected fluorescence having the second polarization.

A presence or an absence of the target nucleic acid molecule in the sample is determined based on the level of anisotropy of the fluorescence (step 908).

For example, in a first case, the nucleic acid molecules do not include the target nucleic acid sequence. In the absence of the target nucleic acid molecules, the DNA polymerase then binds to the reporter molecules, and synthesizes DNA molecules from the reporter molecules. During the synthesis reaction, the DNA polymerase cleaves the fluorophores from the reporter molecules. Light (e.g., polarized light) having a first wavelength then is directed to the mixture. When the fluorophores are excited by the incident light, the fluorophores emit light at a second different wavelength, which can be detected. Due to the relatively high diffusional motion of the unbound fluorophores, the light emitted by the mixture exhibits a relatively low fluorescent anisotropy.

In a second case, the nucleic acid molecules include the target nucleic acid sequence. In this case, the detector nucleic acid molecules hybridize with the target nucleic acid molecules, stabilizing the detector nucleic acid molecules. The resulting hybridized detector nucleic acid molecule binds to the DNA polymerase, and deactivates the enzymatic activity of the DNA polymerase. As a result, DNA synthesis is inhibited, and the fluorophores of the reporter molecules are not cleaved from the reporter molecules. In contrast to the first case, the fluorescent anisotropy of the detected light may be relatively high due, e.g., to the relatively low diffusivity of the still-bound fluorophores.

Accordingly, the presence or the absence of the target nucleic acid molecules can be ascertained by incubating a sample with the reaction solution, inducing fluorescence in the sample, and measuring the anisotropy of the resulting fluorescence

In some cases, the presence or the absence of the target nucleic acid molecule in the sample can be determined by determining that the measured level of anisotropy is greater than or equal to a threshold value. Responsive to determining that the measured level of anisotropy is greater than or equal to the threshold value, a determination can be made that the target nucleic acid molecule is present in the sample.

Further, the presence or the absence of the target nucleic acid molecule in the sample can be determined by determining that the measured level of anisotropy is less than a threshold value. Responsive to determining that the measured level of anisotropy is less than the threshold value, a determination can be made that the target nucleic acid molecule is absent in the sample.

In some cases, a presence of bacteria in the sample can be determined based on the measured level of anisotropy. In some cases, two or more types of bacteria in the sample can be distinguished based on the measured level of anisotropy. In some cases, an indication of cancer can be detected based on the measured level of anisotropy.

In some cases, a sample can be incubated with two or more reaction solutions, such that different target nucleic acid molecules can be detected. For example, the sample can be included with a second reaction solution. The reaction solution can include a plurality of second DNA polymerase molecules, a plurality of second detector nucleic acid molecules, and a plurality of second reporter molecules. Each second detector nucleic acid molecule can include a fourth nucleic acid sequence that is complementary to a fifth nucleic acid sequence of a second target nucleic acid molecule, and a second aptamer that specifically binds to the second DNA polymerase molecules. Each second reporter molecule can include a sixth nucleic acid sequence, a second primer molecule bound to the sixth nucleic acid sequence of the second reporter molecule, and a second fluorophore bound to the sixth nucleic acid sequence of the second reporter molecule.

Each second detector nucleic acid molecule of the plurality of second detector nucleic acid molecules can be configured to hybridize with one or more corresponding second target nucleic acid molecules. Further, each hybridized second detector nucleic acid molecule can be configured to bind to one or more of the second DNA polymerase molecules such that the second DNA polymerase molecules are inactivated.

Second excitation light can be directed to the sample to induce second fluorescence by the second fluorophores. A level of anisotropy of the second fluorescence can be measured, and a presence or an absence of the second target nucleic acid molecule in the sample can be determined based on the level of anisotropy of the second fluorescence.

Some implementations of subject matter and operations described in this specification can be implemented in analog electronic circuitry, digital electronic circuitry, or in computer software, firmware, or hardware, including the structures disclosed in this specification and their structural equivalents, or in combinations of one or more of them. For example, in some implementations, the instruments 200, 400, and/or 500 can be implemented, at least in part, using analog electronic circuitry, digital electronic circuitry, or in computer software, firmware, or hardware, or in combinations of one or more of them. In some implementations, a DAC (e.g., the DAC 204), a measurement assembly (e.g., the measurement assembly 250), an amplifier (e.g., the amplifiers 214), a signal processing module (e.g., the signal processing module 216), a ADC (e.g., the ADCs 218), and/or a microcontroller (e.g., the microcontroller 202) can be implemented, at least in part, using analog electronic circuitry, digital electronic circuitry, or in computer software, firmware, or hardware, or in combinations of one or more of them. In some cases, the process 900 can be performed, at least in part, using digital electronic circuitry, or in computer software, firmware, or hardware, or in combinations of one or more of them.

Some implementations described in this specification can be implemented as one or more groups or modules of analog electronic circuitry, digital electronic circuitry, computer software, firmware, or hardware, or in combinations of one or more of them. Although different modules can be used, each module need not be distinct, and multiple modules can be implemented on the same analog electronic circuitry, digital electronic circuitry, computer software, firmware, or hardware, or combination thereof.

Some implementations described in this specification can be implemented as one or more computer programs, i.e., one or more modules of computer program instructions, encoded on computer storage medium for execution by, or to control the operation of, data processing apparatus. For example, the control application described herein with respect to FIGS. 8A-C) may be implemented as a computer program. A computer storage medium can be, or can be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, disks, or other storage devices).

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

Some of the processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and processors of any kind of digital computer. Generally, a processor will receive instructions and data from a read only memory or a random access memory or both. A computer includes a processor for performing actions in accordance with instructions and one or more memory devices for storing instructions and data. A computer may also include, or be operatively coupled to receive data from or transfer data to, or both, one or more mass storage devices for storing data, e.g., magnetic, magneto optical disks, or optical disks. However, a computer need not have such devices. Devices suitable for storing computer program instructions and data include all forms of non-volatile memory, media and memory devices, including by way of example semiconductor memory devices (e.g., EPROM, EEPROM, flash memory devices, and others), magnetic disks (e.g., internal hard disks, removable disks, and others), magneto optical disks , and CD ROM and DVD-ROM disks. The processor and the memory can be supplemented by, or incorporated in, special purpose logic circuitry.

To provide for interaction with a user, operations can be implemented on a computer having a display device (e.g., a monitor, or another type of display device) for displaying information to the user and a keyboard and a pointing device (e.g., a mouse, a trackball, a tablet, a touch sensitive screen, or another type of pointing device) by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

A computer system may include a single computing device, or multiple computers that operate in proximity or generally remote from each other and typically interact through a communication network. Examples of communication networks include a local area network ("LAN") and a wide area network ("WAN"), an inter-network (e.g., the Internet), a network comprising a satellite link, and peer-to-peer networks (e.g., ad hoc peer-to-peer networks). A relationship of client and server may arise by virtue of computer programs running on the respective computers and having a client-server relationship to each other.

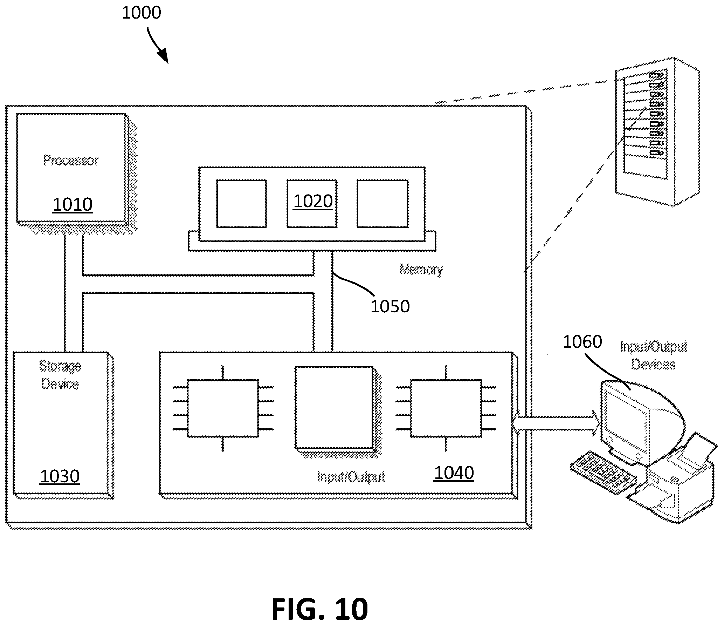

FIG. 10 shows an example computer system 1000 that includes a processor 1010, a memory 1020, a storage device 1030 and an input/output device 1040. Each of the components 1010, 1020, 1030 and 1040 can be interconnected, for example, by a system bus 1050. The processor 1010 is capable of processing instructions for execution within the system 1000. In some implementations, the processor 1010 is a single-threaded processor, a multi-threaded processor, or another type of processor. The processor 1010 is capable of processing instructions stored in the memory 1020 or on the storage device 1030. The memory 1020 and the storage device 1030 can store information within the system 1000.

The input/output device 1040 provides input/output operations for the system 1000. In some implementations, the input/output device 1040 can include one or more of a network interface device , e.g., an Ethernet card, a serial communication device, e.g., an RS-232 port, and/or a wireless interface device, e.g., an 802.11 card, a 3G wireless modem, a 4G wireless modem, etc. In some implementations, the input/output device can include driver devices configured to receive input data and send output data to other input/output devices, e.g., keyboard, printer and display devices 1060. In some implementations, mobile computing devices, mobile communication devices, and other devices can be used.

## Examples of Applications

The implementations described herein can be used in a variety of different applications, including the detection and quantification of various nucleic acid sequences indicative of biological conditions in a subject. In some cases, the detection and quantification of nucleic acid sequences can provide insight into a particular biological or pathogenic process, a particular progression of a particular disease, or some other biological condition.

Examples of various applications are discussed in more detail below and in the Examples section.

Detection of Healthcare-Associated Infections (HAIs):

Healthcare-associated infections (HAIs) and the emergence of drug-resistant pathogens are major healthcare issues. On any given day, 1 out of 25 hospitalized patients becomes infected and as many as 1 out of 9 succumb to death. HAIs incur a significant socioeconomic burden arising from prolonged hospital stays, lasting disability, and demand for new antimicrobials. In the US, it is estimated that more than 600,000 patients develop HAIs every year and HAI-related costs amount to $100 - 150 billion USD per year. Rapid, sensitive detection of pathogenic bacteria can enable the timely initiation of treatment with proper antibiotics, preventing disease spread, and identifying infection sources in hospitals, homes, and other field settings.

One or more of the implementations described herein enable rapid, cost-effective HAI diagnostics. In some implementations, an instrument measures changes in fluorescence anisotropy when detection probes recognize target bacterial nucleic acids. The detection is ratiometric, independent of fluorescence intensity, which makes the assay robust against environmental factors.

Further, some implementations enable multi-level, on-site HAI diagnostics. For example, implementations can include (i) a compact device with a disposable cartridge for sample preparation and multi-well detection, (ii) an assay to perform nucleic acid amplification and detection without washing steps, (iii) embedded contamination control in the assay protocol, (iv) a library of sequence-specific probes to assess bacterial burden, pathogen types, antibiotic resistance, and virulence.

For example, the systems described herein can be used to detect clinically-relevant HAI pathogens including, but not limited to, Gram-negative *Escherichia coli, Klebsiella pneumoniae, Acinetobacter baumannii, Pseudomonas aeruginosa* and Gram-positive *Staphylococcus aureus*. The system may be used to detect sensitivity down to a single bacterium level, determine drug-resistance, and/or determine virulence status. The systems described herein may be used to achieve a detection accuracy comparable to that of bacterial culture. The systems described herein may have a much shorter turnaround time (~2 hours) relative to bacterial cultures and may allow for on-site operation.

Detection of Cancer:

In addition to the detection of pathogenic bacteria, implementations of the system can be expanded to the detection of cancer mutations. To demonstrate, we employed allele-specific polymerase chain reaction to discriminate the cancer mutations and targeted the mutations present in exosomal RNA, which may be more suitable for the longitudinal monitoring of cancer patients than cellular DNA. In addition, to improve the discrimination power, an additional mismatch was introduced at the second base from the 3' end of allele-specific primers. Using this strategy combined with system, we successfully profiled the cancer mutations in exosomal RNA. Further, the exosomal mutation status was confirmed well correlated with the one obtained in cellular genomic DNA.

Examples

The invention is further described in the following examples, which do not limit the scope of the invention described in the claims.

Example 1 - HAI Detection:

*Fabrication of RNA Extraction Device:*

The device was made in plastic via injection molding. A metal block of the mold was first machined to have surface structures such as channels, chambers, and ports negatively shaped to those on the top and the bottom parts of the plastic cartridge. To confine microbeads in the RNA capture chamber, a weir-shaped physical barrier was designed at the outlet side of the chamber. The top and the bottom parts of the device were injection-molded in a foundry (Korea Institute of Machinery & Materials). More than two devices were produced per minute. The top and bottom parts were glued together, and the three-way valve was inserted. Fluidic connection was made by inserting polyethylene tubes on the fluidic ports.

*Preparation of RNA Extraction Device:*

The fluidic cartridge was filled with glass beads (~30 $\mu$m diameter; Polysciences). Beads were suspended in ethanol (75%; Sigma), and introduced through the inlet. The beads were retained in the RNA capture chamber due to the weir-style physical barrier in the outlet side of the chamber (see FIGS. 7A and 7B). Following the bead capture, excess ethanol was collected and removed. The entire device was then flushed with cycles of RNaseZap (Life Technologies), RNase-free water (Life Technologies) and ethanol, and dried. All fluidic flow was generated by manually operating syringes.

*Detection Instrument:*

The illumination source in the optical cube included a light emitting diode (LED; $\lambda = 470$ nm; Thorlabs), a dichroic film polarizer (polarization efficiency >99%; Thorlabs), and a convex lens (focal length $f = 8$ mm). The detection part had a convex lens ($f = 8$ mm), a long-pass filter (EL0500; Thorlabs), a dichroic film polarizer, and a photodiode (S1223, Hamamatsu). A 16-bit digital-to-analog convertor (LTC1597; Linear Technology) was used to deliver the modulated control signal to a custom-designed LED driver (LF356, Texas Instrument). The signal from the photodiode was amplified by a custom-designed current amplifier (AD549; Analog Devices). For the lock-in detection, the amplified signal was passed through a bandpass filter (center frequency, 1kHz; bandwidth, 100 Hz), mixed with a carrier signal, and passed through a lowpass filter (time constant, 1 ms). The conditioned signal was digitized by a 16-bit analog-to-digital converter (LTC1867; Linear Technology). A microcontroller (Arduino MEGA 2560) was programmed to control the light sources for multiplexing, perform real-time data recording, and communicate with an external device (computers, smartphones) via a USB 2.0 or a Bluetooth interface. The entire system was powered by a 9V battery housed inside the base station. A typical power consumption during a single optical measurement (one cube) was ~400 mW, and each test took -30 sec.

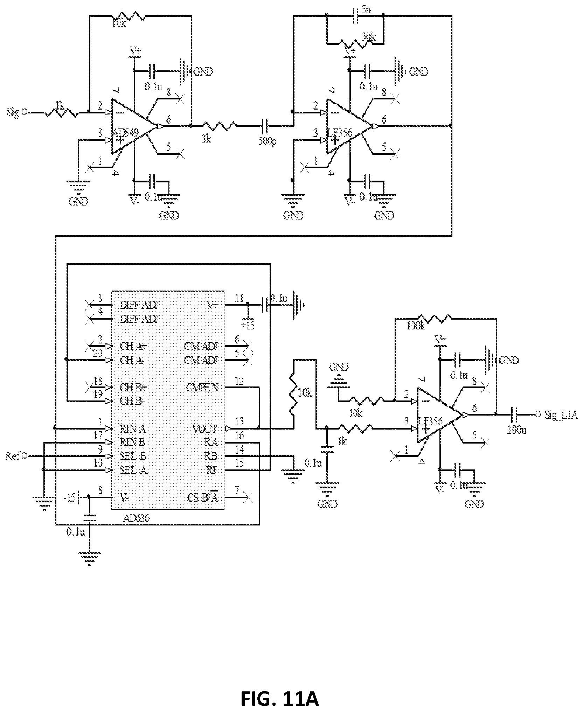

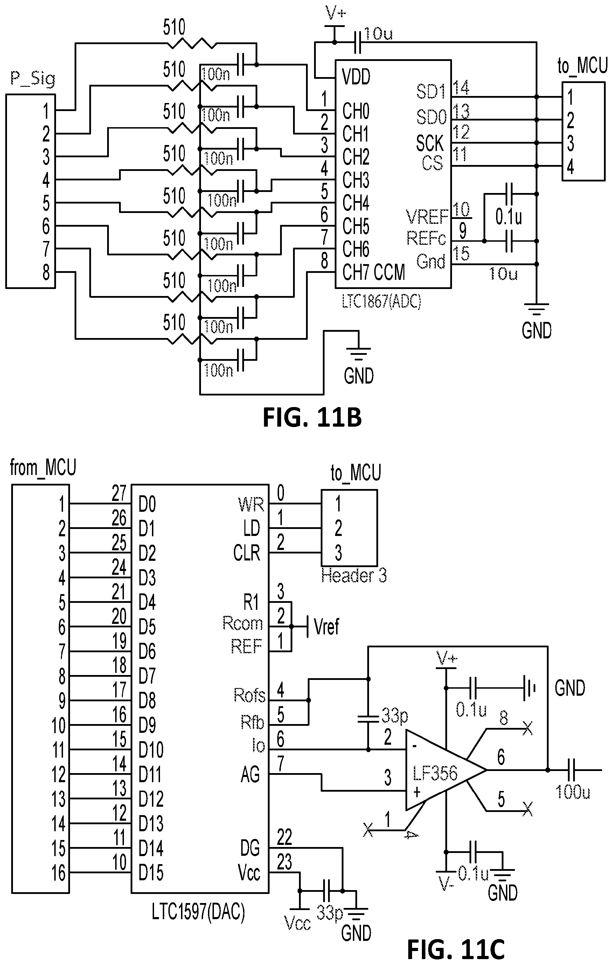

FIG. 11A shows an example current amplifier circuit (AD549; Analog Devices), an active band pass filter, an analog lock-in circuit (AD630; Analog Devices), and a low pass filter. FIG. 11B shows an example 16-bit analog-to-digital converter (LTC1867; Linear Technology) with a configurable 8-channel analog input multiplexer (MUX). FIG. 11C shows an example 16-bit digital-to-analog convertor (LTC1597; Linear Technology) to deliver a modulated control signal to the LED driver.

*Smartphone App:*

We created a custom-designed Android App to facilitate the system operation and data recording. Control software was designed using MIT App Inventor 2. The App connected a smartphone with the system, and sent the triggering signal for the fluorescence anisotropy detection. The measured data were sent back to the phone, and combined with a time stamp and GPS coordinates.

*Signal detection*

The fluorescence anisotropy values were measured with the excitation and emission wavelengths of 470 nm and 525 nm, respectively. The fluorescence anisotropy (r) was calculated using the following equation

$$ r = \left( I_x - I_y \right)\left( I_x + 2I_y \right)^{-1} $$

where $I_x$ and $I_y$ were emission intensities when the emission polarizers were in parallel with and perpendicular to the excitation polarizer, respectively. The limit of detection (LOD) was estimated by setting the threshold at $3\times$ standard deviation (s.d.) above the background signal of samples without bacteria. For the comparison with the benchtop equipment (Sapphire 2; Tecan), we measured $\Delta r = r - r_{FAM}$, where $r_{FAM}$ was the fluorescence anisotropy only in the presence of FAM-DNA (62.5 nM), and $r$ was the fluorescence anisotropy in the presence of FAM-DNA (62.5 nM) along with its template. We varied the template concentrations (10, 20, 30, 40, 50 and 60 nM) to produce different amounts of hybridized FAM-DNA.

*Probe design:*

DNA oligonucleotides were synthesized by Integrated DNA Technologies. The list of DNA sequences are summarized in Tables 1, 2, 3, and 4.

Table 1. Sequence of detection keys designed for the system.

| Category | Target | DNA sequence (5' - 3') |
|---|---|---|
| UNI | Universal | CAATGTACAGTATTGTGGAGCATGTGGTTTAATTCGA |

(continued)

| Category | Target | DNA sequence (5' - 3') |
|---|---|---|
| HAI | *Escherichia* | CAATGTACAGTATTGTGGAGGAAGGGAGTAAAGTTAAT |
| | *Klebsiella* | CAATGTACAGTATTGTGGAGGCAAGGCGATAAGGT |
| | *Acinetobacter* | CAATGTACAGTATTGTTCTAGTTAATACCTAGGGATAGTG |
| | *Pseudomonas* | CAATGTACAGTATTGTGGAGGAAGGGCAGTAAGTTAA |
| | *Staphylococcus* | CAATGTACAGTATTGTGGGAAGAACATATGTGTAAGTAAC |
| ARV | *nuc* | CAATGTACAGTATTGTTGCTTCAGGACCATATTTCTCTAC |
| | *femB* | CAATGTACAGTATTGTCCAGAAGCAAGGTTTAGAATTG |
| | *mecA* | CAATGTACAGTATTGTCTGCTATCCACCCTCAAACAG |
| | PVL | CAATGTACAGTATTGTCGGTAGGTTATTCTTATGGTGGAG |

Table 2. Target sequences recognized by established detection keys.

| Category | Target | DNA sequence (5' - 3') |
|---|---|---|
| Gram-positive | *Streptococcus* | GAAGAACGAGTGTGAGAGTGGAAAGTTCACACTGTGACGGTATCTTACCAGAAA GGGACGGCTAACTA |
| | *Enterococcus* | GAAGAACAAGGACGTTAGTAACTGAACGTCCCCTGACGGTATCTAACCAGAAAG CCACGGCTAACTAC |
| | *Clostridium* | GTCTTCAGGGACGATAATGACGGTACCTGAGGAGGAAGCCACGGCTAACTACG TGCCAGCAGCCGCGGTAAT |
| | *Lactobacillus* | GGAGGCAGCAGTAGGGAATCTTCCACAATGGACGCAAGTCTGATGGAGCAA |
| | *Corynebacterium* | CCTTTCGCAACCGACGAAGCTTTTGTGACGGTAGGTTGAGAAGAAGCACCGGC TAACT |
| | *Bacillus* | GTTGTTAGGGAAGAACAAGTGCGTTCAAATAGGGCGGCACCTTGACGGTACCT AACCAGAAAGCCACGG |
| Gram-negative | *Haemophilus* | TCATGGCATGCGGCCTTGCGGTCCCGCACTTTCATCTTCCGATTCTACGCGGTA TTAGCGAC |
| | *Proteus* | GGAGGAAGGTGATAAGGTTAATACCCTTNTCAATTGACGTTACCCGCAGAAGAA GCACCGGCTAACTCC |
| | *Citrobacter* | ACGGAGTTAGCCGGTGCTTCTTCTGCGAGTAACGTCAATTGCTGCGGTTATTAA CCACAACACCTTCCTCC |
| | *Serratia* | TCAGCGGGGAGGAAGGTGGTGAACTTAATACGTTCATCAATTGACGTTACTCGC AGAAGAAGCACCGGCTAA |
| | *Stenotrophomonas* | AATGCGTAGAGATCAGGAGGAACATCCATGGCGAAGGCAGCTACCTGGACCAA CATTGA |
| | *Legionella* | TTCAGTGGGGAGGAGGATTGATAGGTTAAGAGCTGATTGATTGGACGTTACCCA CAGAAGAAGCACCGGCT |
| | *Neisseria* | TTTGTCAGGGAAGAAAAGGCTGTTGCTAATATCAGCGGCTGATGACGGTACCTG AAGAATAAGCACCGGCTA |
| | *Moraxella* | TTAAGTGGGGAGGAAAAGCTTGTGGTTAATACCCACAAGCCCTGACGTTACCCA CAGAATAAGCA |
| | *Bacteroides* | TCATTAGACATAAAGTGCAGTCATGTCATGTCATACTGTTTTGTCATGTCATAATA TGAATAAGGATCGGCT |
| | *Mycobacterium* | TCTTTCACCATCGACGAAGGTCCGGGTTCTCTGGATTGACGGTAGGTGGAGAA GAAGCACCGGCCAA |

| | Gene Protein name | DNA sequence (5' - 3') . |
|---|---|---|
| ramA | Transcriptional activator | TACCGACCAGCGGGTTTATGATATCTGCCTGAAATACGGCTTTGATTCGCAGCAGAC |
| KPC | Carbapenem-hydrolyzing beta-lactamase | CGGCATAGTCATTTGCCGTGCCATACCCTCCGCAGGTTCCGGTTTTGTCTCCGACTG |
| NDM | Beta-lactamase | CGCATTGGCATAAGTCGCAATCCCCGCCGCATGCAGCGCGTCCATACCGCCCATCTTGT |

16

EP 3 432 785 B1

(continued)

| Gene | Protein name | DNA sequence (5' - 3') . |
|---|---|---|
| OXA | Beta-lactamase | ACCCAACCTGTTAACCAACCTACTTGAGGGGTTACAGCCATTCCCAGCCGCGCTTTTTG |
| IMP | Beta-lactamase | TGAATATTTAGCTTGTGTACCTTACCGGATTTTTTCAAAAGTTCATTTGTTAATTCAGAT |
| VIM | Beta-lactamase | TAGCCGAGGTAGAGGGGAACGAGATTCCCACGCACTCTCTAGAAGGACTCTCATCGAGCG G |
| SPM | Beta-lactamase | CGTTAAAATGCACGGGTTGGGGATGTGAGACTACAGTCTCATTTCGCCAACGGCCTTTTC |
| CTX-M | Beta-lactamase | CGTTAAACACCGCCATTCCGGCGATCCGCGTGATACCCACTTCACCTCGGGC |
| TEM | Beta-lactamase | TTGCACAACATGGGGGATCATGTAACTCGCCTTGATCGTTGGGAACCGGAGCTGAATGAA |
| ampC | Beta-lactamase | GCGTCAAAATCGACACTACCCGACACATGTTGAGTTTTATT CATGCCAACCTTAACCCACAGA |
| vanA | Vancomycin/teic oplanin A-type resistance | TCCAATTCGTCCGCGCTATTGACTTTTTTCACACCGAAGGATGAGCCTGAAC |

Table 3. Summary of a set of antibiotic-resistance and virulence factors targeted in this example.

| Gene | Protein name | Function | Positive strain |
|------|--------------|----------|-----------------|
| *nuc* | Thermonuclease | *S. aureus* specific factor important for in vivo survival | HA-MRSA (ATCC BAA-1720), CA-MRSA (ATCC BAA-1707), MSSA (ATCC 25923) |
| *femB* | Aminoacy ltransferase | *S. aureus* specific factor essential for methicillin resistance, affecting level of resistance | HA-MRSA (ATCC BAA-1720), CA-MRSA (ATCC BAA-1707), MSSA (ATCC 25923) |
| *mecA* | Penicillin binding protein 2A | Methicillin resistance determinant | HA-MRSA (ATCC BAA-1720), CA-MRSA (ATCC BAA-707) |
| PVL | Panton-Valentine Leukocidin | A cytotoxin, one of the β-pore-forming toxins, affecting virulence of *S. aureus* | CA-MRSA (ATCC BAA-1707), MSSA (ATCC 25923) |

| Target | | Strand name | DNA sequence (5' - 3') |
|---|---|---|---|
| Species differentiation | Universal | Amplicon | GGTAAGGTTCTTCGCGTTGCN*TCGAATTAAACCACATGCTCCA |
| | | Limiting primer | GGAGCATGTGGTTTAATTCGA |
| | | Excess primer | GGTAAGGTTCTTCGCGTT |
| | Reporter | Template | CATGAATCTACTCGACGATATTGCTCAACTGTCATAAACTTCTGAGGA |
| | | Primer | TCCTCAGAAGTTT |
| | | FAM-DNA | (FAM)-TATCGTCGAGTAGATTCATG |
| | Escherichia | Amplicon | TGCGGGTAACGTCAATGAGCAAAGGTATTAACTTTACTCCCTTCCTCCA |
| | | Limiting primer | GGAGGAAGGGAGTAAAGTTAATACCTTTG |
| | | Excess primer | TGCGGGTAACGTCAATGAG |
| | Klebsiella | Amplicon | ACGGCAGTTAGCCGGTGCTTCTTCTGCGGGTAACGTCCAATCGCCAAGGTTATTAACCTTATCGCCTTGCCTCCA |
| | | Limiting primer | GGAGGCAAGGCGATAAGGT |
| | | Excess primer | ACGGCAGTTAGCCGGTGCTTCT |
| | Acinetobacter | Amplicon | GAGTTAGCCGGTGCTTATTCTGCGAGTAACGTCCACTATCCCTAGGTATTAACTAGAA |
| | | Limiting primer | TCTAGTTAATACCTAGGGATAGTGGACGTT |
| | | Excess primer | GAGTTAGCCGGTGCTTATTCTGCGAG |
| | Pseudomonas | Amplicon | TATTCTGTTGGTAACGTCAAAACAGCAAGGTATTAACTTACTGCCCTTCCTCCA |
| | | Limiting primer | GGAGGAAGGGCAGTAAGTTAATACCTTG |
| | | Excess primer | GCTTATTCTGTTGGTAACGTCAAAACAG |
| | Staphylococcus | Amplicon | GTAGTTAGCCGGTGGCTTTCTGATTAGGTACCGTCAAGATGTGCACAGTTACTTACACATATGTTCTTCCCA |
| | | Limiting primer | GGGAAGAACATATGTGTAAGTAAC |
| | | Excess primer | GTAGTTAGCCGGTGGCTTTCT |
| Antibiotic-resistance and virulence | nuc | Amplicon | CACCTGAAACAAAGCATCCTAAAAAAGGTGTAGAGAAATATGGTCCTGAAGCAA |
| | | Limiting primer | TGCTTCAGGACCATATTTCTCTAC |
| | | Excess primer | CACCTGAAACAAAGCATCCTAAA |
| | femB | Amplicon | TGAATTGAGCAAAACGGACGGCCCAATTCTAAACCTTGCTTCTGGA |
| | | Limiting primer | CCAGAAGCAAGGTTTAGAATTG |
| | | Excess primer | TGAATTGAGCAAAACGGACGGC |
| | mecA | Amplicon | ATGAAGGTGTGCTTACAAGTGCTAATAATTCACCTGTTTGAGGGTGGATAGCAGA |
| | | Limiting primer | CTGCTATCCACCCTCAAACAGG |
| | | Excess primer | ATGAAGGTGTGCTTACAAGTGC |
| | PVL | Amplicon | CACCTGATAAGCCGTTAGAGATATTAATATCTCCACCATAAGAATAACCTACCGA |
| | | Limiting primer | CGGTAGGTTATTCTTATGGTGGAGAT |
| | | Excess primer | CACCTGATAAGCCGTTAGAGATATT |

Table 4. DNA sequences used in this example. The nucleobase in bold letter (N) indicates Adenine (A) for *Escherichia, Klebsiella, Acinetobacter* or Thymine (T) for *Pseudomonas, Staphylococcus.*

For the universal and species-specific detection of pathogenic bacteria, individual 16S rRNA sequences of different bacterial genera (from the NCBI nucleotide database) were aligned using MegAlign software (DNAStar), and both conserved and variable regions were selected as target sequences. For the detection of antibiotic-resistance and virulence factors, the specific regions of nuc, femB, mecA and Panton-Valentine Leukocidin (PVL) (from the NCBI nucleotide database) were selected as target sequences. The detection keys were designed to have a hairpin structure joined by a single-stranded capture sequence (20 ~ 25 nucleotides in length).

*Lyophilization of master-mix:*

All-in-one PAD-mix (20 μL) was concocted by mixing a detection key (400 nM), a reporter (150 nM), primer (150 nM) and FAM-labeled DNA (125 nM) in 20 mM Tris-HCl (pH 8.3), 20 mM KCl, 5 mM (NH4)2SO4, and 6 mM MgCl2. The mixture was first frozen in liquid nitrogen and then dried in Virtis Freezemobile 25EL Freeze Dryer (SP scientific). The lyophilized reagents were stored at room temperature, and prior to its use, was reconstituted by adding 20 μL of Ultra pure DNase/RNase-free distilled water (Life Technologies).

*Experiment with cultured bacteria:*

All bacteria were purchased from the American Type Culture Collection (ATCC). Bacterial cultures were grown to mid-log phase in vendor-recommended media: *E. coli* (#25922) in Luria-Bertani media (BD Biosciences); *P. aeruginosa* (#142), *K. pneumoniae* (#43816), methicillin-resistant *S. aureus* (#BAA-1720 and #BAA-1707) in tryptic soy broth (BD Biosciences); *A. baumannii* (#15149) in nutrient broth (BD Biosciences); *S. aureus* (#25923) in Staphylococcus broth (BD Biosciences). Bacteria were collected via centrifugation (6000 g, 10 min), and pellets were resuspended with the pre-heated Trizol (Life Technologies). The resuspended cells were transferred to 2 mL Safe-Lock tubes (Eppendorf) containing sterilized disruptor beads (0.1 mm, Scientific Industries), and lysed using a vortex mixer. After centrifugation, the supernatant was transferred to a new tube.

*RNA extraction*

Bacteria lysate mixed with an equal volume of ethanol was flown through the RNA extraction chamber, where RNA was captured by packed glass beads. Subsequent flushing with Direct-zol RNA PreWash (Zymo Research) and RNA Wash Buffer (Zymo Research) was used to remove traces of impurities and chaotropic salts. Finally, RNA was eluted in RNase-free water. For comparison of the fluidic cartridge with a commercial column (Zymo-SpinTM Column, Zymo Research), bacteria lysate was divided into two aliquots. One sample was processed by the commercial column, and the other by the fluidic cartridge. We checked the quality of the extracted RNA through an electrophoretic assay (Bioanalyzer, 2100; Agilent). RNA molecular weight ladder provided in the kit (RNA 6000 nano chip; Agilent) was used as reference and electrophoretic runs were performed per manufacturer's protocol. The analysis assigned RNA-integrity-numbers (RINs) to samples, ranging from one to ten, where one indicates highly degraded RNA and ten completely intact RNA.

*System assay:*

The single-stranded cDNA was synthesized using random priming with Promega's Reverse Transcription System per manufacturer's protocol. The asymmetric PCR amplification was then carried out in a total reaction volume of 25 μL containing 2.5 μL of cDNA, 0.8 μM of excess primer and 0.08 μM of limiting primer (see Table 4), 1 × PCR reaction buffer (20 mM Tris-HCl, 20 mM KCl, 5 mM (NH4)2SO4, and 2 or 3 mM MgCl2), 0.2 mM of dATP, dGTP and dCTP, 0.4 mM of dUTP (Thermo Scientific), 2 U antarctic thermolabile UDG (New England Biolabs) and 2.5 U Maxima Hot Start Taq DNA polymerase (Thermo Scientific). For the asymmetric PCR on a miniaturized thermocycler (MiniPCR; Amplyus), we used the following cycling condition: 25 °C for 10 min and 94 °C for 4 min; 35 cycles of 30 s at 94 °C, 30 s at 56 °C, and 30 s at 72 °C; and an extension step 10 min at 72 °C. With a benchtop thermocycler (MasterCycler; Eppendorf), the reaction condition was 25 °C for 10 min and 94 °C for 4 min; 35 cycles of 5 s at 94 °C, 15 s at 56 °C, and 15 s at 72 °C; and a final 10 min at 72 °C. 20 μL of the PCR solution was mixed with all-in-one PAD-mix composed of detection key (200 nM) and reporter that was pre-formed with template (75 nM), primer (75 nM) and FAM-labeled DNA (62.5 nM) at room temperature for 15 min, making a total volume of 40 μL in 20 mM Tris-HCl (pH 8.3), 20 mM KCl, 5 mM (NH4)2SO4, and 4 mM MgCl2. UDG-mediated control of carryover contamination

To mimic the carryover contamination, we spiked dUTP-containing amplification products (carryover contaminants) into new reaction samples. The copy number of carryover contaminants was ~107; a single aerosol after PCR reaction typically contains as many as 106 amplification products(26). The dUTP-containing amplicons were prepared following the same procedures outlined above except that equal amount of limiting and excess primers was used. The obtained amplicons were purified using Zymoclean Gel DNA recovery kit (Zymo Research) and quantified by measuring the absorbance at 260 nm with Nanodrop 1000 (Thermo Scientific). The copy number was estimated based on the conversion factor, 26 kDa/amplicon.

*Clinical samples:*

This study was approved by the Partners Institutional Review Board. Excess and discarded samples were collected from nine subjects with clinical suspicion for infected bodily fluid or abscess and referred for drainage. Specimens were collected using routine image guided approaches by MGH Interventional Radiology physicians and analyzed blindly via the PAD assay. Specimens (500 μL) were mixed with 1.5 mL of Trizol LS (Life Technologies) that is more concentrated than Trizol, and the same RNA extraction procedure was applied as in pure bacterial cultures.

*Electrophoretic band shift experiment*

Solution containing 100 or 200 nM detection key, PCR products and 12.5 U Taq DNA polymerase (New England Biolabs) in 20 mM Tris-HCl (pH 8.3), 20 mM KCl, 5 mM (NH4)2SO4, and 4 mM MgCl2 was incubated at room temperature for 20 min. The solution was mixed with 6× loading buffer and subjected to electrophoresis on a 20 % polyacrylamide gel (Life technologies). The analysis was carried out in 1× TBE (100 mM Tris, 90 mM Borate, 1 mM EDTA) at 150 V for 160 min at 4 °C. After GelRed (Biotium) staining, gels were scanned using a UV transilluminator. DNA polymerase was neither fluorescent nor stained by the dye.

*Quantitative real-time PCR:*

The cDNA derived from in-vitro cultured bacteria or clinical samples were mixed with 1x SYBR Select Master mix (Life Technologies) and 0.4 µM specific primers used in the PAD assay. Thermal cycling was then carried out on 7500 Fast Real-Time PCR system (Life Technologies) with the following conditions: UDG activation (50 °C, 2 min), initiation (95 °C, 2 min); 40 cycles of denaturation (95 °C, 5 s); annealing (56 °C, 15 s); extension (72 °C, 30 s). The 7500 Fast software automatically calculates the Ct value, which represents the first PCR cycle at which the fluorescence signal exceeds the signal of a given uniform threshold. No-template control (NTC) remained undetected, not crossing the established threshold for 40 cycles, which was arbitrarily given a *Ct* value of 41. The $\Delta Ct$ was generated by subtracting the Ct value of the specimen from the Ct value of NTC(28).

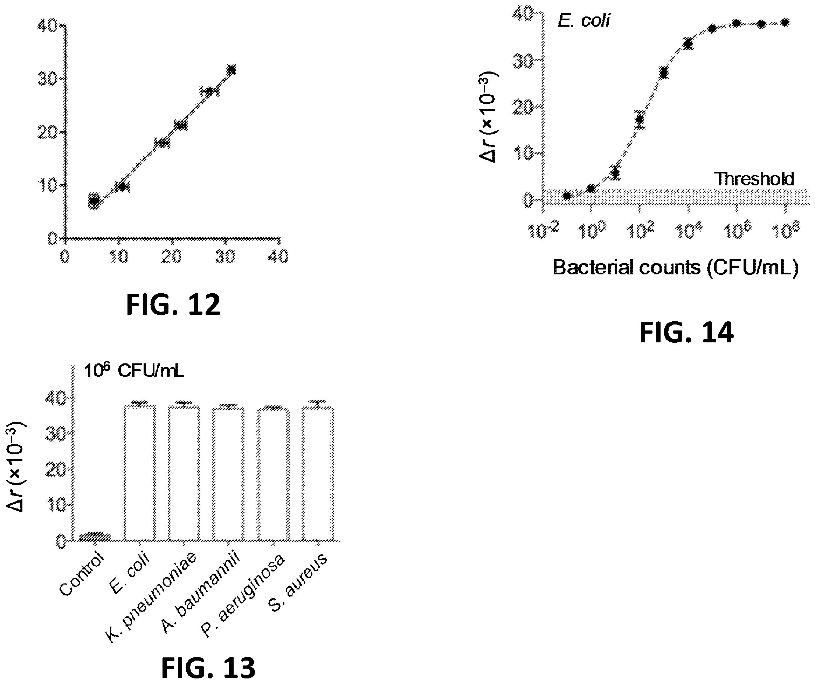

In this example, we benchmarked the system against a commercial plate-reader (Sapphire 2, Tecan). To prepare samples of different fluorescence anisotropy, we varied the ratio between free fluorescein-labeled DNA (FAM-DNA; high fluorescence anisotropy) and template-bound FAM-DNA (low anisotropy). The observed r values showed an excellent agreement ($R_2 > 99\%$) between the plate-reader and the system, which served to confirm the accuracy of the system, as shown in FIG. 12.

*Universal key to measure bacterial load*

In some applications, a "universal detection key" can be designed for detecting multiple different biological particles (e.g., pathogens). That is, the system can be used with a single detection key that is capable of detecting a particular target nucleic acid sequence common across multiple different biological particles. An example of a single, universal key (UNI key) designed to target a conserved region of 16S rRNA in different bacterial species is depicted in Table 1 (see also FIG. 1B).

Table 1. Sequence of detection keys designed for the system.

| Category | arget T | DNA sequence (5' - 3') |
|---|---|---|
| UNI | Universal | CAA TGT ACAGT A TTGTGGAGCA TGTGGTTT AA TTCGA |
| HAI | *Escherichia* | CAA TGT ACAGT A TTGTGGAGGAAGGGAGT AAAGTT AAT |
| | *Klebsiella* | CAATGTACAGTATTGTGGAGGCAAGGCGATAAGGT |
| | *Acinetobacter* | CAATGTACAGTATTGTTCTAGTTAATACCTAGGGATAGTG |
| | *Pseudomonas* | CAATGTACAGTATTGTGGAGGAAGGGCAGTAAGTTAA |
| | *Staphylococcus* | CAATGTACAGTATTGTGGGAAGAACATATGTGTAAGTAAC |
| ARV | *nuc* | CAATGTACAGTATTGTTGCTTCAGGACCATATTTCTCTAC |
| | *femB* | CAATGTACAGTATTGTCCAGAAGCAAGGTTTAGAATTG |
| | *mecA* | CAATGTACAGTATTGTCTGCTATCCACCCTCAAACAG |
| | PVL | CAATGTACAGTATTGTCGGTAGGTTATTCTTATGGTGGAG |

In some cases, it is also possible to design a common reporter probe. For instance, a common reporter probe can be used for a variety of different detection targets. An exemplary common reporter probe that includes FAM-DNA, its primer, and template is shown in FIG. 1B. The system output was defined as $\Delta r = r - r_0$, where $r_0$ is the fluorescence anisotropy of control samples containing DNA polymerase and the reporter only.

We applied the designed assay (UNI-PAD) to detect representative HAI pathogens (*E. coli, K. pneumoniae, A. baumannii, P. Aeruginosa,* and *S. aureus*). Across different bacterial species, we observed consistent $\Delta r$ values in concentration-matched samples (see FIG. 13, one-way ANOVA, P = 0.8857). As shown in FIG. 13, give different HAI pathogens (106 CFU/mL) were detected with the system. The signal levels were statistically identical among concentration-matched bacterial samples. This result supported the use of the UNI-PAD in estimating total bacterial load.

We next performed titration experiments with serially diluted bacterial samples (see FIG. 14). As shown in FIG. 14, samples with different bacterial concentrations (*E. coli,* $10^{-1}$-$10^8$ CFU/mL) were prepared through serial dilution. The limit of detection was at the level of single bacterium. CFU, colony forming unit. The threshold was set at $3\times$ s.d. above background signal of the sample without bacteria. The assay achieved the dynamic range spanning >104 colony forming units (CFUs). The limit of detection was down to single-digit CFUs.

All experiments were performed in triplicate, and the graphs are displayed as mean $\pm$ s.d.

*Assay for point-of-care operation:*

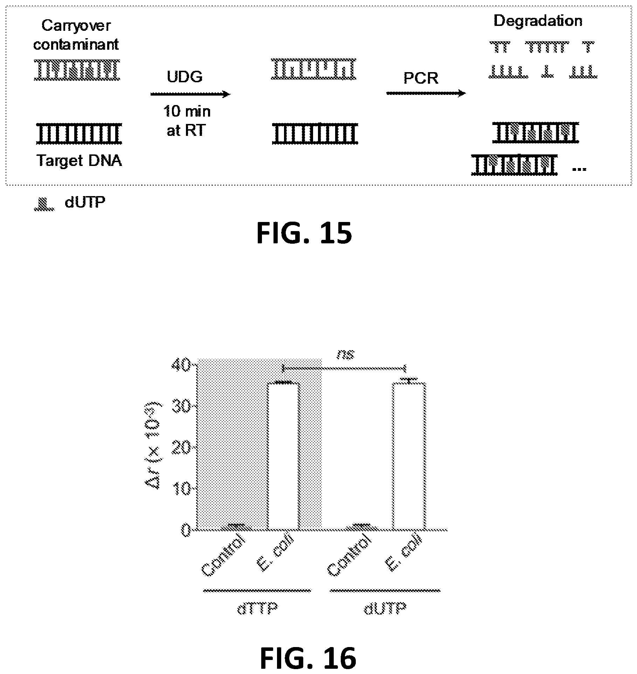

The systems described herein also may be used for point-of-care (POC) applications. One major issue in POC nucleic acid testing is to control false positives caused by sample contamination with PCR products (e.g., carryover contamination). To minimize such effects, we adopted the uracil-DNA glycosylase (UDG) method. By substituting deoxythymidine triphosphate (dTTP) with deoxyuridine triphosphate (dUTP) during PCR, we rendered all amplicons to have an uracil-containing DNA backbone. Applying UDG cleaved these amplicons, which selectively destroyed carryover contaminants from PCR reactions, while keeping bona fide DNA templates (see FIG. 15). We confirmed that the method was compatible with the assay. The signal level remained the same when dUTP replaced dTTP in DNA targets (see FIG. 16). As shown in FIG. 16, as compared to the original assay condition in which dTTP was used (left), no difference was observed when dTTP was replaced with dUTP (right). The observed $\Delta r$ values in both cases were statistically identical (two-tailed t-test, P > 0.99).

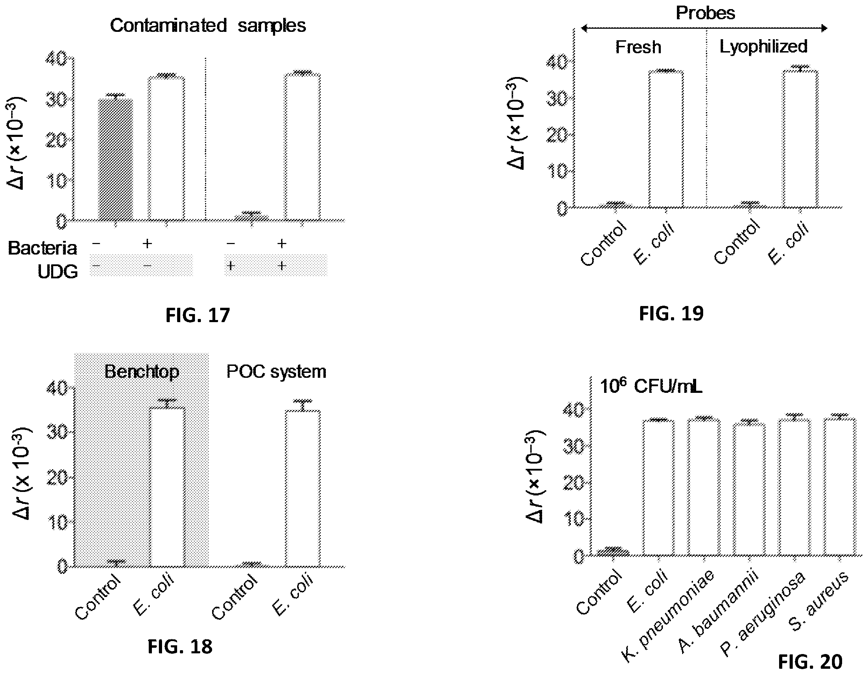

We next tested the efficacy of this contamination control. As a carryover contaminant, dUTP-containing PCR products (107 amplicons) were spiked into samples. Without UDG treatment, the negative control with no bacterial targets showed false positives (see FIG. 17). This signal was eliminated with the addition of UDG, and only samples containing true bacterial targets yielded high $\Delta r$ (see FIG. 17).

To maximize the system portability, we further combined the system with a miniaturized thermocycler (MiniPCR; Ampylus). The performance of the POC system matched with that of a conventional bench-top equipment (see FIG. 18). As shown in FIG. 18, the performance of a benchtop equipment and the POC-PAD was statistically identical (two-tailed t-test, P > 0.69).

We also lyophilized all chemical reagents (e.g., detection keys, reporters) to facilitate their transport and storage. The reagents retained their activities after >2 weeks of storage in ambient condition. We observed statistically identical $\Delta r$ values (two-tailed t-test, P > 0.64) with fresh and stored agents (see FIGS. 19 and 20). All experiments were performed in triplicate, and the data are displayed as mean $\pm$ s.d.

*Differential keys for pathogen classification:*

In some cases, a set of different detection keys (HAI keys) can be designed for differentiating HAI-causing pathogens. In an example, each key may be designed to target the hypervariable region of 16S rRNA in different bacterial species. Examples of bacterial species for which differential detection keys have been designed are identified in Table 2.

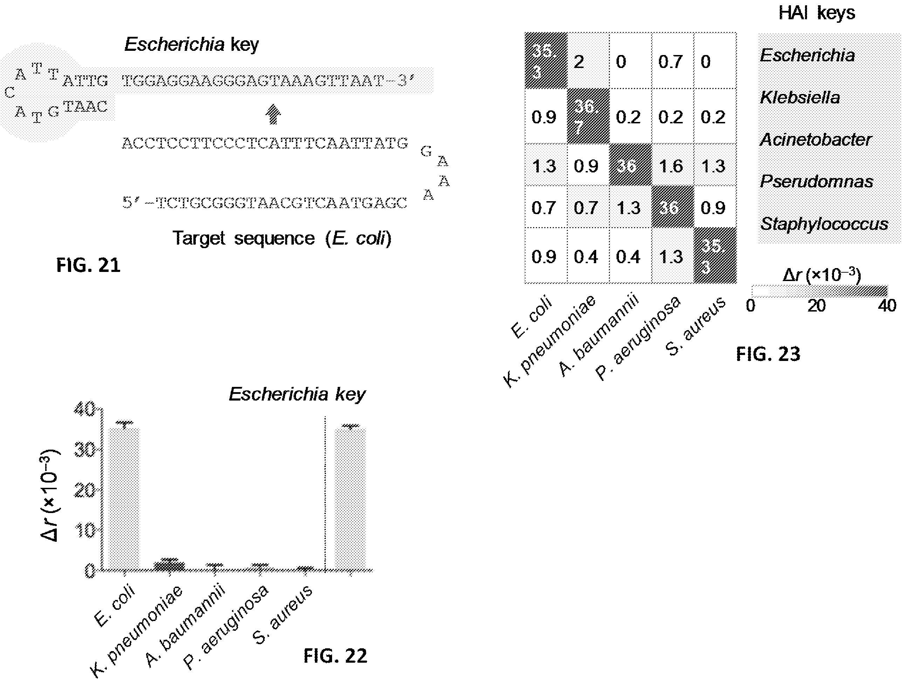

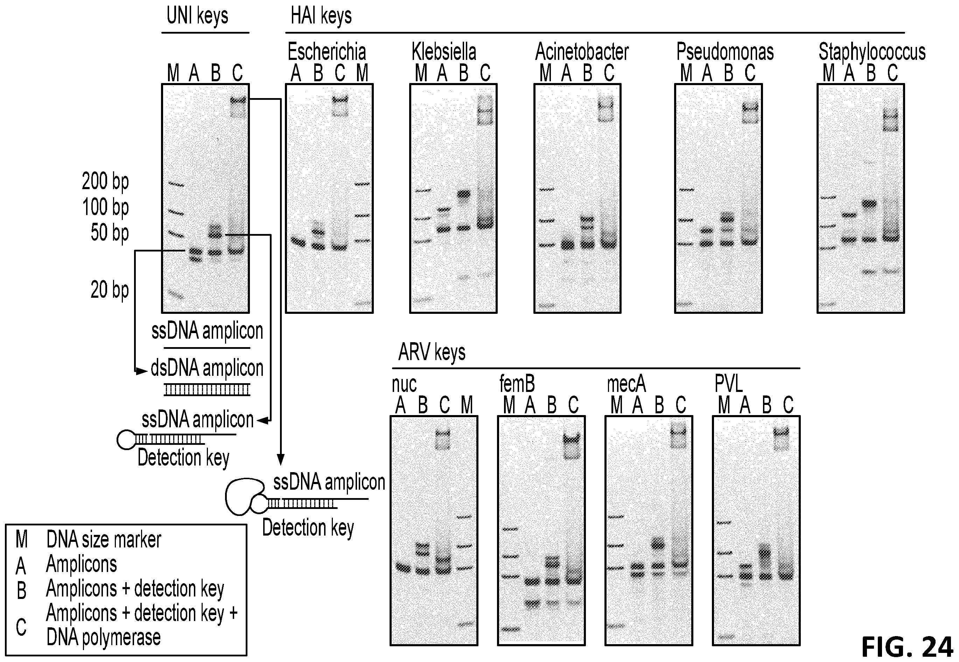

The sequence homology among genus types was kept <50% to minimize nonspecific binding. When tested, the HAI keys assumed high specificity. For example, the *Escherichia* key (see FIG. 21) showed high signal ($\Delta r$) only with its intended target, whereas off-target signals were negligible even in high biological background (106 CFU of other bacterial species, see FIG. 22). Similarly, other HAI keys displayed an excellent specificity with minimal crosstalk (see FIG. 23). Electrophoretic band-shift analysis confirmed that the detection keys, in the presence of complementary target amplicon, bound to DNA polymerase and inhibited its catalytic activity (see FIG. 24). As shown in FIG. 24, Lane A: amplicons only, Lane B: binding of amplicons with their detection keys produced new bands, Lane C: adding DNA polymerase to B shifted the band for the amplicon and detection-key hybrid. This confirmed the binding between detection keys and DNA polymerase.

We next prepared probes for antibiotic-resistance and virulence (ARV keys) for further bacterial phenotyping. These keys targeted bacterial genes that make pathogens antibiotic-resistant or highly virulent. As a model system, we profiled samples for *mecA*, PVL, *nuc,* and *femB* genes. *mecA* is the determinant factor conferring methicillin resistance to *S. aureus* (MRSA), a common multidrug-resistant HAI pathogen. PVL, *nuc,* and *femB* are virulence factors that contribute to the pathogenicity of *S. aureus.* We used two representative MRSA strains which have the following known genotypes: healthcare-associated MRSA (*mecA*+, PVL-) and community-acquired MRSA (*mecA*+, PVL+). Control samples were methicillin-sensitive *S. aureus* (MSSA) (*mecA*-), *E. coli* (*mecA-,* PVL-, *nuc-, femB*-), and *P. aeruginosa* (*mecA-,* PVL-, *nuc-, femB-*) (see Table 3).

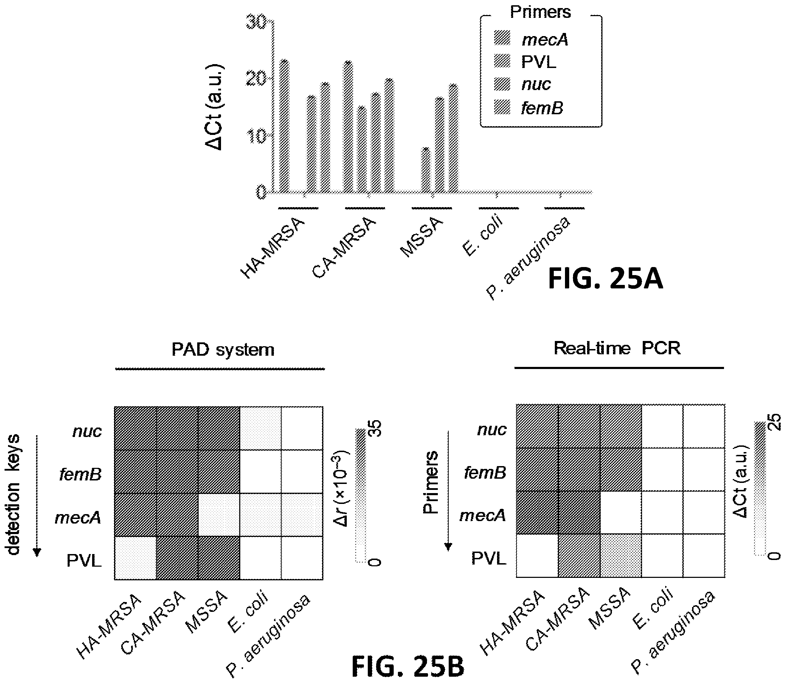

The ARV-PAD correctly genotyped bacteria, agreeing well with a quantitative real-time PCR (qPCR) (see FIGS. 24, 25A, and 25B). As shown in FIG. 25A, real-time PCR detection of *mecA*, PVL, *nuc,* and *femB* in bacterial species was performed. SYBR Green assay was used. A comparison between the PAD and the qPCR assays is shown in FIG. 25B.

*Clinical application:*

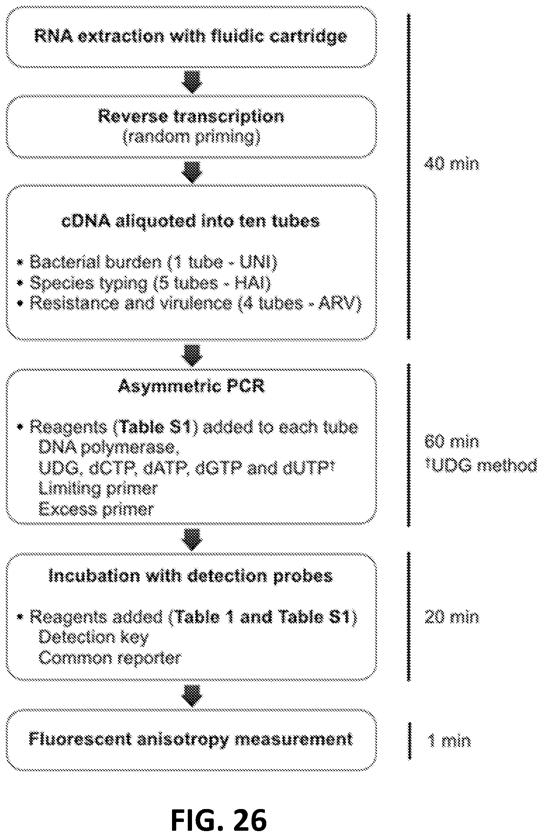

The systems described herein can also be used for clinical HAI diagnostics. For example, the systems described herein can be used to identify the presence of particular HAI pathogens, total bacterial burden (e.g., using a universal key), or antibiotic resistance/virulence status. The assay started with bacterial lysis, followed by the nucleic acid collection using the plastic cartridge. Following the nucleic acid amplification, the samples were analyzed in parallel for the total bacterial burden (UNI key), HAI pathogens (HAI keys), and antibiotic-resistance / virulence status (ARV keys) (see FIG. 26). The total assay time was ~ 2 hours and the required sample volume was ~ 40 μL.

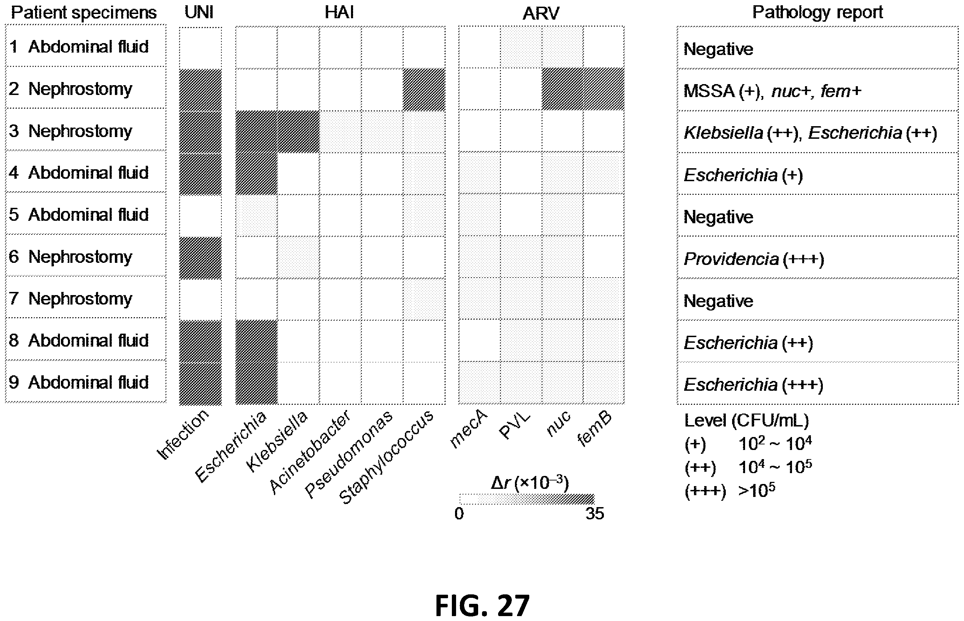

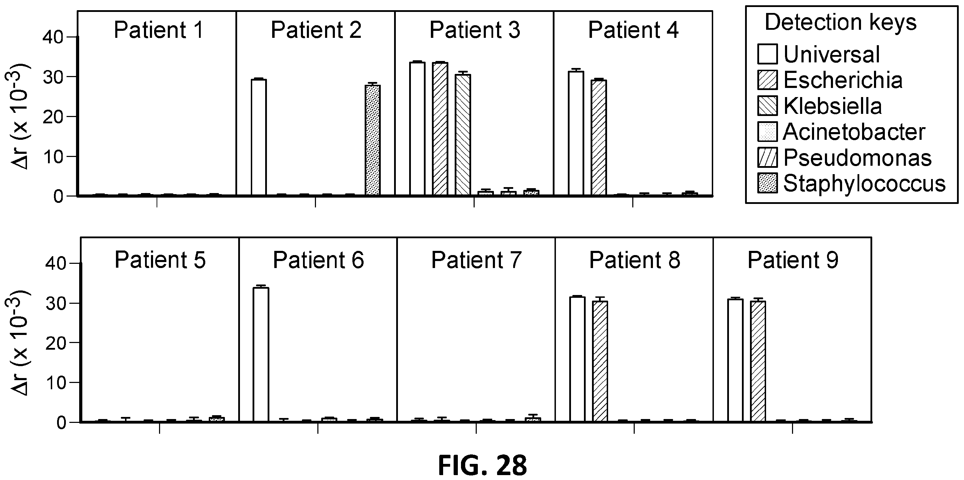

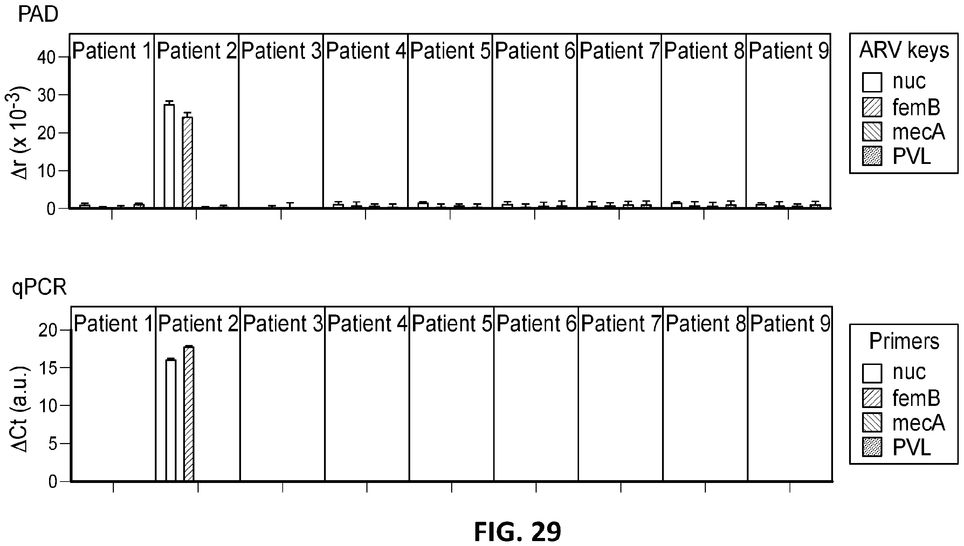

We acquired patient samples, and aliquoted them for system test (2 hours) and conventional culture (3-5 days) in a clinical microbiology laboratory. Test results for all detection keys (UNI, HAI, ARV) are shown in FIGS. 27 and 28. As shown in FIG. 27, nine samples from different patients were processed by the system for the bacterial load (UNI), the presence of the HAI species (HAI), and the resistance/virulence status (ARV). The clinical samples were also tested by a clinical pathology laboratory (culture and qPCR). The PAD and the pathology reports agreed each other. Samples negative with UNI-PAD were also negative with HAI-PAD, suggesting the potential use of universal detection for sample triaging. Among six UNI-PAD positive samples, the HAI-PAD detected HAI pathogens in five samples, and the differentiation results matched the bacterial culture readouts. One patient (No. 6) was positive with bacterial load, but was negative with HAI keys. That patient was later found to be infected with *P. Rettgeri* (non-targeted in the current HAI-PAD). For the sample positive for *S. aureus* (Patient No. 2), the ARV-PAD showed *mecA*- status (i.e., MSSA); this result matched the MRSA-negative pathology report and qPCR (see FIGS. 28 and 29).

*Discussion:*

Healthcare-associated infections (HAIs) have become a ubiquitous, recalcitrant and costly problem in modern healthcare. HAIs increase the emergence of antibiotics-resistance, cause significant morbidity and mortality, and prolong hospital stays. One of the key mandates to control this endemic is to equip local hospitals and community centers with more effective surveillance systems. Implementations of the system described herein could enable rapid HAI detection in those settings. The detection system is compact and user-friendly with minimal operation complexity. The assay is comprehensive, assessing for overall bacterial burden, pathogen types, antibiotic resistance, and virulence factors.

Several features make the system ideal for field operation. First, the sensing scheme (fluorescence anisotropy) is inherently robust against environmental noise. We further incorporated the optical lock-in technique to significantly enhance the sensitivity. Second, the assay flow involves minimal complexity and hands-on time. A disposable plastic chip is used to collect nucleic acids, and the remaining processes are performed in a single tube without washing steps. The assay protocol is also refined to automatically dissolve carryover contaminants, thereby minimizing false positives. Third, the platform is highly affordable. The system has relatively simple electronics, and can readily be expanded for parallel detection. Making injection-molded cartridge brings advantages including high performance reproducibility, less cross-contamination between samples, and lower cost. Finally, the system is scalable for comprehensive screening. Decoupling detection and signaling probes enables such assays cost-effective, because a common fluorescence reporter can be used for all detection targets. We have already designed detection probes for >35 targets (see Table 2); the incremental assay cost is relatively low.

In a pilot clinical test, the system accuracy was comparable to that of bacterial culture. In contrast to the culture, the system assay was fast (~2 hours), multiplexed, and cost-effective.

In some cases, the system can be a self-contained, closed system, and sample preparation, thermocycling, and detection functions can all be housed in a single device. Such a system can effectively eliminate erroneous results from sample contamination, user-interference or both, and proffer "sample-in and answer-out" tests. In some cases, assay time could be shortened, particularly for DNA amplification. For example, a photonic thermocycling system can be used, which can complete the entire PCR reaction in <5 min. Isothermal amplification also can be used. Some isothermal reactions can be completed in 20 min, which could bring down the total assay time to <1 hour. Employing isothermal amplification can simplify the hardware requirement, and can enable a battery-powered device. Further, the test library can be expected for broader pathogen and antibiotic-resistance screening. The modular nature of system probes can facilitate incorporating additional targets such as host-response factors (e.g. interleukin-4, platelet-derived growth factor-B chain, monocyte chemoattractant protein-1, and C-X-C motif chemokine 10)(23), as well as other viral, fungal and parasitic markers.

Example 2 - Detection of Cancer:

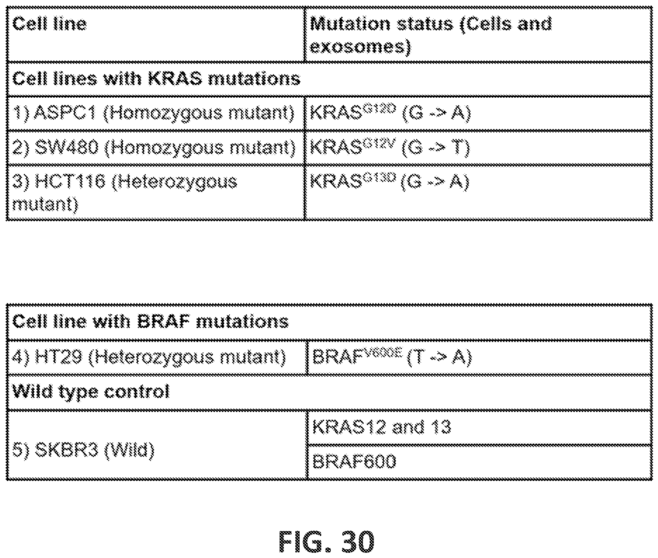

FIG. 30 summaries the cells used in this example, and their respective mutation statuses. For the detection of cancer mutations, allele-specific PCR was employed to discriminate cancer mutation. The key of allele-specific PCR is the primer whose 3' end is perfectly matched only in the mutant samples. But, a potential limitation of the allele-specific PCR technique is the low discrimination power, which can lead to false positive signals due to the erroneous amplification in the wild type samples.

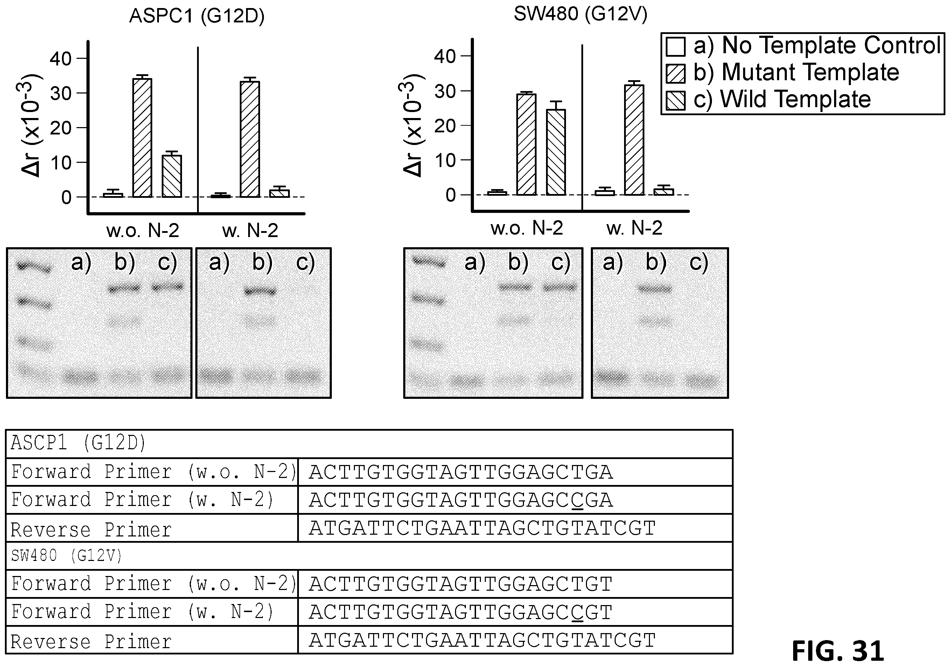

To improve this low discrimination power, an additional mismatch at the N-2 position was introduced in the primer. As shown in FIG. 31, it was observed that when the primers contained an additional N-2 mismatch, the erroneous amplification in the wild type samples was significantly suppressed without interfering with the amplification reactions in the mutant samples (G12D and G12V). This dramatically increased the discriminative power of ASPCR by greatly diminishing nonspecific amplification.

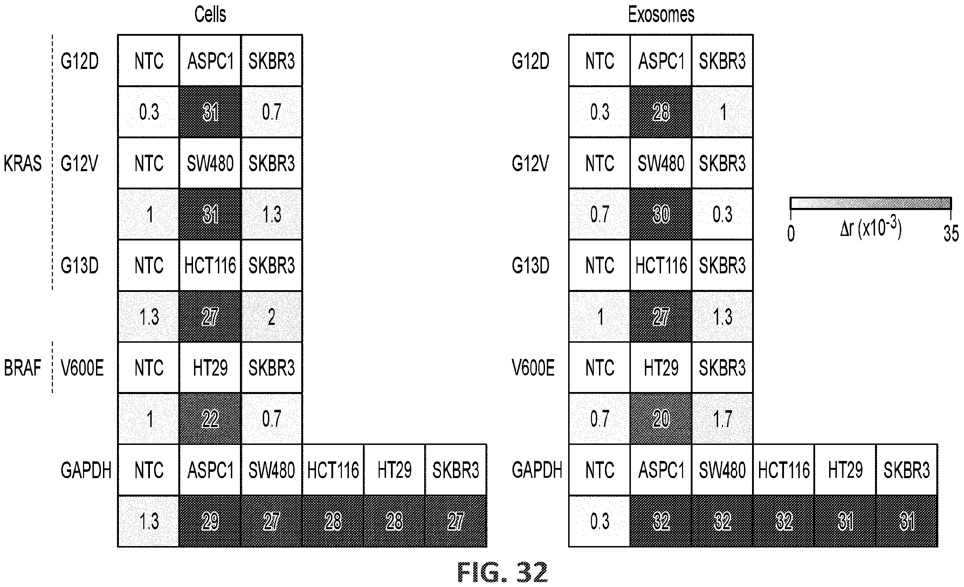

This modified allele-specific PCR technique was employed in PAD system. As explained, the amplification products were produced only in the presence of mutant samples, which consequently induced the high fluorescence anisotropy values. Based on this strategy, mutation status in cancer cells and exosomes were profiled. FIG. 32 shows the profiling of somatic mutations in cells (DNA amount equivalent to 5 x $10^4$ cells) and their secreting exosomes (cDNA amount equivalent to $10^9$ exosomes). As expected, only in the mutant samples (ASPC1, SW480, HCT116, and HT29) exhibited the high fluorescence anisotropy values, while the wild type sample (SKBR3) that did not produce the amplification products showed the low fluorescence anisotropy values. GAPDH was used as the housekeeping gene that is constantly expressed in all the tested cells. As shown in FIG. 31, to improve specificity, an additional mismatch was introduced at the second base from the 3' end of allele-specific primers. This dramatically increased the discriminative power of ASPCR by greatly diminishing nonspecific amplification.

FIG. 32 shows a profiling of somatic mutations in cells (DNA amount equivalent to 5 x $10^4$ cells) and their secreting exosomes (cDNA amount equivalent to $10^9$ exosomes)

## Claims

1. A method of detecting a first target nucleic acid molecule and a second target nucleic acid molecule in a sample, the method comprising:

   (a) incubating a sample with a first reaction solution and a second reaction solution, wherein the first reaction solution comprises:

   (i) a plurality of first DNA polymerase molecules,
   (ii) a plurality of first detector nucleic acid molecules, wherein each first detector nucleic acid molecule comprises a first nucleic acid sequence that is complementary to a second nucleic acid sequence of a first target nucleic acid molecule, and a first aptamer that specifically binds to the first DNA polymerase molecules, and
   (iii) a plurality of first reporter molecules, wherein each first reporter molecule comprises a third nucleic acid sequence, a first primer molecule bound to the third nucleic acid sequence of the first reporter molecule, and a first fluorophore bound to the third nucleic acid sequence of the first reporter molecule,

   wherein each first detector nucleic acid molecule of the plurality of first detector nucleic acid molecules is configured to hybridize with one or more corresponding first target nucleic acid molecules, wherein, for each first reporter molecule of the plurality of first reporter molecules, the plurality of first DNA polymerase molecules are configured to cleave the first fluorophore from the first reporter molecule, and
   wherein each first hybridized detector nucleic acid molecule is configured to bind to one or more of the first DNA polymerase molecules such that the first DNA polymerase molecules are inactivated, wherein inactivating a first DNA polymerase molecule inhibits cleaving of the first fluorophore from the first reporter molecule, and

   wherein the second reaction solution comprises:

   (i) a plurality of second DNA polymerase molecules,
   (ii) a plurality of second detector nucleic acid molecules, wherein each second detector nucleic acid molecule comprises a fourth nucleic acid sequence that is complementary to a fifth nucleic acid sequence of a second target nucleic acid molecule, and a second aptamer that specifically binds to the second DNA polymerase molecules, and
   (iii) a plurality of second reporter molecules, wherein each second reporter molecule comprises a sixth nucleic acid sequence, a second primer molecule bound to the sixth nucleic acid sequence of the second reporter molecule, and a second fluorophore bound to the sixth nucleic acid sequence of the second reporter molecule,
wherein each second detector nucleic acid molecule of the plurality of second detector nucleic acid molecules is configured to hybridize with one or more corresponding second target nucleic acid molecules, wherein, for each second reporter molecule of the plurality of second reporter molecules, the plurality of second DNA polymerase molecules are configured to cleave the second fluorophore from the second reporter molecule, and
wherein each hybridized second detector nucleic acid molecule is configured to bind to one or more of the second DNA polymerase molecules such that the second DNA polymerase molecules are inactivated, wherein inactivating a second DNA polymerase molecule inhibits cleaving of the second fluorophore from the second reporter molecule;

(b) directing first excitation light to the sample to induce first fluorescence by the first fluorophores, wherein directing the first excitation light to the sample comprises:

generating a first carrier signal having a first frequency,
generating the first excitation light based on the first carrier signal;

(c) measuring a level of anisotropy of the first fluorescence, wherein measuring the level of anisotropy of the first fluorescence comprises:

measuring a first photodetector signal of a photodetector in optical communication with the sample,
filtering the first photodetector signal using a first band pass filter having the first frequency as a center frequency to obtain a first filtered signal,
mixing the first filtered signal with the first carrier signal to obtain a first mixed signal,
filtering the first mixed signal using a low pass filter to obtain a first output signal, and
determining level of anisotropy of the first fluorescence based on the first output signal;

(d) determining a presence or an absence of the first target nucleic acid molecule in the sample based on the level of anisotropy of the first fluorescence;
(e) directing second excitation light to the sample to induce second fluorescence by the second fluorophores, wherein directing the second excitation light to the sample comprises:

generating a second carrier signal having a second frequency,
generating the second excitation light based on the second carrier signal;

(f) measuring a level of anisotropy of the second fluorescence, wherein measuring the level of anisotropy of the second fluorescence comprises:

measuring a second photodetector signal of a photodetector in optical communication with the sample,
filtering the second photodetector signal using a second band pass filter having the first frequency as a center frequency to obtain a first filtered signal,
mixing the second filtered signal with the first carrier signal to obtain a second mixed signal,
filtering the second mixed signal using a low pass filter to obtain a second output signal, and
determining level of anisotropy of the second fluorescence based on the second output signal; and

(g) determining a presence or an absence of the second target nucleic acid molecule in the sample based on the level of anisotropy of the second fluorescence,

wherein at least one of:

the first excitation light comprises a first wavelength of light, and wherein the second excitation light comprises a second wavelength of light different from the first wavelength of light, or
the first fluorescence comprises a first wavelength of light, and wherein the second fluorescence comprises a second wavelength of light different from the first wavelength of light.

2. The method of claim 1, wherein directing first excitation light to the sample comprises directing first polarized light to the sample, and/or wherein measuring the level of anisotropy of the first fluorescence comprises:

detecting first fluorescence having a first polarization;
detecting first fluorescence having a second polarization that is different than the first polarization; and
determining the level of anisotropy of the first fluorescence based on the detected first fluorescence having the first polarization and the detected first fluorescence having the second polarization.

3. The method of claim 1, wherein determining the presence or the absence of the first target nucleic acid molecule in the sample comprises:

determining that the measured level of anisotropy of the first fluorescence is greater than or equal to a first threshold value; and
responsive to determining that the measured level of anisotropy of the first fluorescence is greater than or equal to the first threshold value, determining that the first target nucleic acid molecule is present in the sample, and/or wherein determining the presence or the absence of the first target nucleic acid molecule in the sample comprises:

determining that the measured level of anisotropy of the first fluorescence is less than a first threshold value; and
responsive to determining that the measured level of anisotropy of the first fluorescence is less than the first threshold value, determining that the first target nucleic acid molecule is absent in the sample.

4. The method of claim 1, wherein at least one of the first target nucleic acid molecule or the second target nucleic acid molecule is a ribonucleic acid (RNA) molecule.

5. The method of claim 1, further comprising determining a presence of bacteria in the sample or an indication of cancer based on the measured level of anisotropy of the first fluorescence, and/or further comprising distinguishing between two or more types of bacteria in the sample based on the measured level of anisotropy of the first fluorescence.

6. A system for detecting a first target nucleic acid molecule and a second target nucleic acid in a sample, the system comprising:

one or more computer processors; and
one or more measurement assemblies communicatively coupled to the one or more computer processors, wherein each measurement assembly comprises:

a detection region configured to accept a sample;
a light assembly configured and controlled by the one or more computer processors to direct first excitation light and second excitation light into the sample in the detection region;
a first photodetector assembly; and
a second photodetector assembly, wherein the first and second photodetector assemblies are configured and controlled by the one or more computer processors to measure a level of anisotropy of first fluorescence and a level of anisotropy of second fluorescence emitted from the sample in the detection region, and

wherein the one or more computer processors is configured to determine a presence or an absence of the first target nucleic acid molecule and the second target nucleic in the sample based on the level of anisotropy of the first fluorescence and the level of anisotropy of the second fluorescence, and
wherein the system is configured to perform the method of any one of claims 1-5.

7. The system of claim 6, wherein the light assembly comprises a light source, and a first polarizer disposed between the light source and the detection region, optionally wherein the first photodetector assembly comprises a first photodiode, and a second polarizer disposed between the first photodiode and the detection region, optionally wherein the second photodetector assembly comprises a second photodiode, and a third polarizer disposed between the second photodiode and the detection region,

8. The system of claim 6, wherein the system comprises a plurality of measurement assemblies, optionally further comprising a heating assembly, wherein the heating assembly is configured, during operation of the system, to apply heat to the detection region of each of the one or more measurement assemblies.

9. The system of claim 6, further comprising a wireless transceiver configured to transmit data and/or receive data from a computing device external to the system, optionally wherein the computer device is a smartphone or a tablet computer.

**10.** The system of claim 6, wherein the light assembly is configured and controlled by the one or more computer processors to:

direct the first excitation light having a first wavelength into the sample in the detection region; and
direct the second excitation light having a second wavelength into the sample in the detection region, wherein the first wavelength is different than the second wavelength, wherein the light assembly is configured and controlled by the one or more computer processors to alternatingly direct the first excitation light having the first wavelength into the sample in the detection region and direct the second excitation light having the second wavelength into the sample in the detection region over a period of time,
optionally wherein the one or more computer processors are enclosed in a housing, and wherein the one or more measurement assemblies are reversibly attachable to the housing via a communications interface.

## Patentansprüche

**1.** Verfahren zum Detektieren eines ersten Zielnukleinsäuremoleküls und eines zweiten Zielnukleinsäuremoleküls in einer Probe, wobei das Verfahren umfasst:

(a) Inkubieren einer Probe mit einer ersten Reaktionslösung und einer zweiten Reaktionslösung, wobei die erste Reaktionslösung umfasst:

(i) eine Vielzahl von ersten DNA-Polymerasemolekülen,
(ii) eine Vielzahl von ersten Detektornukleinsäuremolekülen, wobei jedes erste Detektornukleinsäuremolekül eine erste Nukleinsäuresequenz, die zu einer zweiten Nukleinsäuresequenz eines ersten Zielnukleinsäuremoleküls komplementär ist, und ein erstes Aptamer umfasst, das spezifisch an die ersten DNA-Polymerasemoleküle bindet, und
(iii) eine Vielzahl von ersten Reportermolekülen, wobei jedes erste Reportermolekül eine dritte Nukleinsäuresequenz, ein erstes Primermolekül, das an die dritte Nukleinsäuresequenz des ersten Reportermoleküls gebunden ist, und ein erstes Fluorophor umfasst, welches an die dritte Nukleinsäuresequenz des ersten Reportermoleküls gebunden ist,
wobei jedes erste Detektornukleinsäuremolekül von der Vielzahl der ersten Detektornukleinsäuremoleküle so ausgestaltet ist, dass es nur an ein oder mehrere entsprechende erste Zielnukleinsäuremoleküle hybridisiert,
wobei die Vielzahl der ersten DNA-Polymerasemoleküle für jedes erste Reportermolekül der Vielzahl von ersten Reportermolekülen so ausgestaltet ist, dass das erste Fluorophor von dem ersten Reportermolekül abgespalten wird, und wobei jedes erste hybridisierte Detektornukleinsäuremolekül so ausgestaltet ist, dass es an ein oder mehrere von den ersten DNA-Polymerasemolekülen bindet, so dass die ersten DNA-Polymerasemoleküle inaktiviert werden, wobei Inaktivieren eines ersten DNA-Polymerasemoleküls das Abspalten des ersten Fluorophors von dem ersten Reportermolekül inhibiert, und

wobei die zweite Reaktionslösung umfasst:

(i) eine Vielzahl von zweiten DNA-Polymerasemolekülen,
(ii) eine Vielzahl von zweiten Detektornukleinsäuremolekülen, wobei jedes zweite Detektornukleinsäuremolekül eine vierte Nukleinsäuresequenz, die zu einer fünften Nukleinsäuresequenz eines zweiten Zielnukleinsäuremoleküls komplementär ist, und ein zweites Aptamer umfasst, das spezifisch an die zweiten DNA-Polymerasemoleküle bindet, und
(iii) eine Vielzahl von zweiten Reportermolekülen, wobei jedes zweite Reportermolekül eine sechste Nukleinsäuresequenz, ein zweites Primermolekül, das an die sechste Nukleinsäuresequenz des zweiten Reportermoleküls gebunden ist, und ein zweites Fluorophor umfasst, welches an die sechste Nukleinsäuresequenz des zweiten Reportermoleküls gebunden ist,

wobei jedes zweite Detektornukleinsäuremolekül von der Vielzahl der zweiten Detektornukleinsäuremoleküle so ausgestaltet ist, dass es an ein oder mehrere entsprechende zweite Zielnukleinsäuremoleküle hybridisiert,
wobei die Vielzahl der zweiten DNA-Polymerasemoleküle für jedes zweite Reportermolekül der Vielzahl von

EP 3 432 785 B1

zweiten Reportermolekülen so ausgestaltet ist, dass das zweite Fluorophor von dem zweiten Reportermolekül abgespalten wird, und

wobei jedes hybridisierte zweite Detektornukleinsäuremolekül so ausgestaltet ist, dass es an ein oder mehrere von den zweiten DNA-Polymerasemolekülen bindet, so dass die zweiten DNA-Polymerasemoleküle inaktiviert werden, wobei Inaktivieren eines zweiten DNA-Polymerasemoleküls das Abspalten des zweiten Fluorophors von dem zweiten Reportermolekül inhibiert;

(b) Lenken von erstem Anregungslicht auf die Probe, um erste Fluoreszenz durch die ersten Fluorophore zu induzieren, wobei Lenken des ersten Anregungslichts zu der Probe umfasst:

Generieren eines ersten Trägersignals mit einer ersten Frequenz,
Generieren des ersten Anregungslichtes basierend auf dem ersten Trägersignal;

(c) Messen eines Anisotropieniveaus der ersten Fluoreszenz, wobei Messen des Anisotropieniveaus der ersten Fluoreszenz umfasst:

Messen eines ersten Photodetektorsignals von einem Photodetektor in optischer Kommunikation mit der Probe,
Filtern des ersten Photodetektorsignals unter Verwendung eines ersten Bandpassfilters mit der ersten Frequenz als Mittenfrequenz, um ein erstes gefiltertes Signal zu erhalten,
Mischen des ersten gefilterten Signals mit dem ersten Trägersignal, um ein erstes gemischtes Signal bereitzustellen;
Filtern des ersten gemischten Signals unter Verwendung eines Tiefpassfilters, um ein erstes Ausgabesignal zu erhalten, und
Bestimmen des Anisotropieniveaus der ersten Fluoreszenz basierend auf dem ersten Ausgabesignal;

(d) Bestimmen einer Anwesenheit oder einer Abwesenheit des ersten Zielnukleinsäuremoleküls in der Probe basierend auf dem Anisotropieniveau der ersten Fluoreszenz;

(e) Lenken von zweitem Anregungslicht auf die Probe, um zweite Fluoreszenz durch die zweiten Fluorophore zu induzieren, wobei Lenken des zweiten Anregungslichts zu der Probe umfasst:

Generieren eines zweiten Trägersignals mit einer zweiten Frequenz,
Generieren des zweiten Anregungslichtes basierend auf dem zweiten Trägersignal;

(f) Messen eines Anisotropieniveaus der zweiten Fluoreszenz, wobei Messen des Anisotropieniveaus der zweiten Fluoreszenz umfasst:

Messen eines zweiten Photodetektorsignals von einem Photodetektor in optischer Kommunikation mit der Probe,
Filtern des zweiten Photodetektorsignals unter Verwendung eines zweiten Bandpassfilters mit der ersten Frequenz als Mittenfrequenz, um ein erstes gefiltertes Signal zu erhalten,
Mischen des zweiten gefilterten Signals mit dem ersten Trägersignal, um ein zweites gemischtes Signal bereitzustellen;
Filtern des zweiten gemischten Signals unter Verwendung eines Tiefpassfilters, um ein zweites Ausgabesignal zu erhalten, und
Bestimmen des Anisotropieniveaus der zweiten Fluoreszenz basierend auf dem zweiten Ausgabesignal; und

(g) Bestimmen einer Anwesenheit oder einer Abwesenheit des zweiten Zielnukleinsäuremoleküls in der Probe basierend auf dem Anisotropieniveau der zweiten Fluoreszenz,
wobei mindestens eines der folgenden zutrifft:

das erste Anregungslicht umfasst eine erste Wellenlänge des Lichts, und wobei das zweite Anregungslicht eine zweite Wellenlänge des Lichts umfasst, die sich von der ersten Wellenlänge des Lichts unterscheidet, oder
die erste Fluoreszenz umfasst eine erste Wellenlänge des Lichts, und wobei die zweite Fluoreszenz eine zweite Wellenlänge des Lichts umfasst, die sich von der ersten Wellenlänge des Lichts unterscheidet.

2. Verfahren nach Anspruch 1, wobei Lenken von erstem Anregungslicht auf die Probe Lenken von erstem polarisiertem Licht auf die Probe umfasst, und/oder wobei Messen des Anisotropieniveaus der ersten Fluoreszenz umfasst:

Detektieren von erster Fluoreszenz mit einer ersten Polarisation;
Detektieren von erster Fluoreszenz mit einer zweiten Polarisation, die sich von der ersten Polarisation unterscheidet; und
Bestimmen des Anisotropieniveaus der ersten Fluoreszenz basierend auf der detektierten ersten Fluoreszenz mit der ersten Polarisation und der detektierten ersten Fluoreszenz mit der zweiten Polarisation.

3. Verfahren nach Anspruch 1, wobei Bestimmen der Anwesenheit oder der Abwesenheit des ersten Zielnukleinsäuremoleküls in der Probe umfasst:

Bestimmen, dass das gemessene Anisotropieniveau der ersten Fluoreszenz größer als oder gleich einem ersten Schwellenwert ist; und
in Reaktion darauf, dass bestimmt wird, dass das gemessene Anisotropieniveau der ersten Fluoreszenz größer als oder gleich dem ersten Schwellenwert ist, Bestimmen, dass das erste Zielnukleinsäuremolekül in der Probe vorhanden ist, und/oder wobei Bestimmen der Anwesenheit oder
der Abwesenheit des ersten Zielnukleinsäuremoleküls in der Probe umfasst:

Bestimmen, dass das gemessene Anisotropieniveau der ersten Fluoreszenz kleiner als ein erster Schwellenwert ist; und
in Reaktion auf das Bestimmen, dass das gemessene Anisotropieniveau der ersten Fluoreszenz kleiner als der erste Schwellenwert ist, Bestimmen, dass das erste Zielnukleinsäuremolekül in der Probe abwesend ist.

4. Verfahren nach Anspruch 1, wobei mindestens eines von dem ersten Zielnukleinsäuremolekül oder dem zweiten Zielnukleinsäuremolekül ein Ribonukleinsäure- (RNA)-Molekül ist.

5. Verfahren nach Anspruch 1, des Weiteren umfassend Bestimmen einer Anwesenheit von Bakterien in der Probe oder eines Anzeichens von Krebs basierend auf dem gemessenen Anisotropieniveau der ersten Fluoreszenz, und/oder des Weiteren umfassend Unterscheiden zwischen zwei oder mehr Typen von Bakterien in der Probe basierend auf dem gemessenen Anisotropieniveau der ersten Fluoreszenz.

6. System zum Detektieren eines ersten Zielnukleinsäuremoleküls und einer zweiten Zielnukleinsäure in einer Probe, wobei das System umfasst:

einen oder mehrere Computerprozessoren und
eine oder mehrere Messanordnungen, die kommunikativ mit dem einen oder den mehreren Computerprozessoren gekoppelt sind, wobei jede Messanordnung umfasst:

eine Detektierungsregion, die ausgestaltet ist, um eine Probe anzunehmen;
eine Lichtanordnung, die ausgestaltet ist und durch den einen oder die mehreren Computerprozessoren gesteuert wird, um erstes Anregungslicht und zweites Anregungslicht in die Probe in der Detektierungsregion zu lenken;
eine erste Photodetektoranordnung; und
eine zweite Photodetektoranordnung, wobei die erste und die zweite Photodetektoranordnung ausgestaltet sind und durch den einen oder die mehreren Computerprozessoren gesteuert werden, um ein Anisotropieniveau der ersten Fluoreszenz und ein Anisotropieniveau der zweiten Fluoreszenz zu messen, die von der Probe in der Detektierungsregion emittiert werden, und
wobei der eine oder die mehreren Computerprozessoren ausgestaltet ist, um eine Anwesenheit oder eine Abwesenheit des ersten Zielnukleinsäuremoleküls und der zweiten Zielnukleinsäure in der Probe basierend auf dem Anisotropieniveau der ersten Fluoreszenz und dem Anisotropieniveau der zweiten Fluoreszenz zu bestimmen, und
wobei das System ausgestaltet ist, um das Verfahren nach einem der Ansprüche 1 bis 5 durchzuführen.

7. System nach Anspruch 6, wobei die Lichtanordnung eine Lichtquelle umfasst und ein erster Polarisator zwischen der Lichtquelle und der Detektierungsregion angeordnet ist, wobei gegebenenfalls die erste Photodetektoranordnung eine erste Photodiode umfasst, und ein zweiter Polarisator zwischen der ersten Photodiode und der Detektierungsregion angeordnet ist, wobei gegebenenfalls die zweite Photodetektoranordnung eine zweite Photodiode umfasst, und wobei ein dritter Polarisator zwischen der zweiten Photodiode und der Detektierungsregion angeordnet ist.

8. System nach Anspruch 6, wobei das System eine Vielzahl von Messanordnungen umfasst, gegebenenfalls des Weiteren eine Heizanordnung umfasst, wobei die Heizanordnung ausgestaltet ist, um während des Betriebs des Systems Wärme auf die Detektierungsregion von jeder der einen oder mehreren Messanordnungen anzuwenden.

9. System nach Anspruch 6, des Weiteren umfassend einen drahtlosen Sendeempfänger, der ausgestaltet ist, um Daten von einer Rechenvorrichtung außerhalb des Systems zu übertragen und/oder Daten von dieser zu empfangen, wobei gegebenenfalls die Computervorrichtung ein Smartphone oder ein Tablet-Computer ist.

10. System nach Anspruch 6, wobei die Lichtanordnung ausgestaltet ist und durch den einen oder die mehreren Computerprozessoren so gesteuert wird, dass sie folgendes ausführt:

Lenken des ersten Anregungslichts mit einer ersten Wellenlänge in die Probe in der Detektierungsregion; und Lenken des zweiten Anregungslichts mit einer zweiten Wellenlänge in die Probe in der Detektierungsregion, wobei die erste Wellenlänge sich von der zweiten Wellenlänge unterscheidet, wobei die Lichtanordnung ausgestaltet ist und durch den einen oder die mehreren Computerprozessoren so gesteuert wird, dass alternierend über einen Zeitraum das erste Anregungslicht mit der ersten Wellenlänge in die Probe in der Detektierungsregion gelenkt wird und das zweite Anregungslicht mit der zweiten Wellenlänge in die Probe in der Detektierungsregion gelenkt wird,
wobei gegebenenfalls der eine oder die mehreren Computerprozessoren in ein Gehäuse eingeschlossen sind, und wobei die eine oder mehreren Messanordnungen über eine Kommunikationsschnittstelle reversibel an dem Gehäuse befestigbar sind.

**Revendications**

1. Procédé de détection d'une première molécule d'acide nucléique cible et d'une seconde molécule d'acide nucléique cible dans un échantillon, le procédé comprenant :

(a) l'incubation d'un échantillon avec une première solution réactionnelle et une seconde solution réactionnelle, la première solution réactionnelle comprenant :

(i) une pluralité de premières molécules d'ADN polymérase,
(ii) une pluralité de premières molécules d'acide nucléique détectrices, chaque première molécule d'acide nucléique détectrice comprenant une première séquence d'acide nucléique qui est complémentaire d'une deuxième séquence d'acide nucléique d'une première molécule d'acide nucléique cible, et un premier aptamère qui se lie spécifiquement aux premières molécules d'ADN polymérase, et
(iii) une pluralité de premières molécules rapporteuses, chaque première molécule rapporteuse comprenant une troisième séquence d'acide nucléique, une première molécule d'amorce liée à la troisième séquence d'acide nucléique de la première molécule rapporteuse, et un premier fluorophore lié à la troisième séquence d'acide nucléique de la première molécule rapporteuse,
chaque première molécule d'acide nucléique détectrice de la pluralité de premières molécules d'acide nucléique détectrices étant configurée pour s'hybrider avec une ou plusieurs premières molécules d'acide nucléique cibles correspondantes, pour chaque première molécule rapporteuse de la pluralité de premières molécules rapporteuses, la pluralité de premières molécules d'ADN polymérase étant configurées pour cliver le premier fluorophore de la première molécule rapporteuse, et
chaque première molécule d'acide nucléique détectrice hybride étant configurée pour se lier à une ou plusieurs des premières molécules d'ADN polymérase de telle sorte que les premières molécules d'ADN polymérase sont inactivées, l'inactivation d'une première molécule d'ADN polymérase inhibant le clivage du premier fluorophore de la première molécule rapporteuse, et

la seconde solution réactionnelle comprenant :

(i) une pluralité de secondes molécules d'ADN polymérase,
(ii) une pluralité de secondes molécules d'acide nucléique détectrices, chaque seconde molécule d'acide nucléique détectrice comprenant une quatrième séquence d'acide nucléique qui est complémentaire d'une cinquième séquence d'acide nucléique d'une seconde molécule d'acide nucléique cible, et un second aptamère qui se lie spécifiquement aux secondes molécules d'ADN polymérase, et

(iii) une pluralité de secondes molécules rapporteuses, chaque seconde molécule rapporteuse comprenant une sixième séquence d'acide nucléique, une seconde molécule d'amorce liée à la sixième séquence d'acide nucléique de la seconde molécule rapporteuse, et un second fluorophore lié à la sixième séquence d'acide nucléique de la seconde molécule rapporteuse,

chaque seconde molécule d'acide nucléique détectrice de la pluralité de secondes molécules d'acide nucléique détectrices étant configurée pour s'hybrider avec une ou plusieurs secondes molécules d'acide nucléique cibles correspondantes, pour chaque seconde molécule rapporteuse de la pluralité de secondes molécules rapporteuses, la pluralité de secondes molécules d'ADN polymérase étant configurées pour cliver le second fluorophore de la seconde molécule rapporteuse, et

chaque seconde molécule d'acide nucléique détectrice hybride étant configurée pour se lier à une ou plusieurs des secondes molécules d'ADN polymérase de telle sorte que les secondes molécules d'ADN polymérase sont inactivées, l'inactivation d'une seconde molécule d'ADN polymérase inhibant le clivage du second fluorophore de la seconde molécule rapporteuse ;

(b) l'orientation d'une première lumière d'excitation vers l'échantillon pour induire une première fluorescence par les premiers fluorophores, l'orientation de la première lumière d'excitation vers l'échantillon comprenant :

la génération d'un premier signal porteur ayant une première fréquence,
la génération de la première lumière d'excitation sur la base du premier signal porteur ;

(c) la mesure d'un niveau d'anisotropie de la première fluorescence,
la mesure du niveau d'anisotropie de la première fluorescence comprenant :

la mesure d'un premier signal de photodétecteur d'un photodétecteur en communication optique avec l'échantillon,
le filtrage du premier signal de photodétecteur en utilisant un premier filtre passe-bande ayant la première fréquence comme fréquence centrale pour obtenir un premier signal filtré,
le mélange du premier signal filtré avec le premier signal porteur pour obtenir un premier signal mélangé,
le filtrage du premier signal mélangé en utilisant un filtre passe-bas pour obtenir un premier signal de sortie, et
la détermination du niveau d'anisotropie de la première fluorescence sur la base premier signal de sortie ;

(d) la détermination d'une présence ou d'une absence de la première molécule d'acide nucléique cible dans l'échantillon sur la base du niveau d'anisotropie de la première fluorescence ;

(e) l'orientation d'une seconde lumière d'excitation vers l'échantillon pour induire une seconde fluorescence par les seconds fluorophores, l'orientation de la seconde lumière d'excitation vers l'échantillon comprenant :

la génération d'un second signal porteur ayant une seconde fréquence,
la génération de la seconde lumière d'excitation sur la base du second signal porteur ;

(f) la mesure d'un niveau d'anisotropie de la seconde fluorescence, la mesure du niveau d'anisotropie de la seconde fluorescence comprenant :

la mesure d'un second signal de photodétecteur d'un photodétecteur en communication optique avec l'échantillon,
le filtrage du second signal de photodétecteur en utilisant un second filtre passe-bande ayant la première fréquence comme fréquence centrale pour obtenir un premier signal filtré,
le mélange du second signal filtré avec le premier signal porteur pour obtenir un second signal mélangé,
en filtrant le second signal mélangé en utilisant un filtre passe-bas pour obtenir un second signal de sortie, et
la détermination du niveau d'anisotropie de la seconde fluorescence sur la base du second signal de sortie ;
et

(g) la détermination d'une présence ou d'une absence de la seconde molécule d'acide nucléique cible dans l'échantillon sur la base du niveau d'anisotropie de la seconde fluorescence,
au moins un parmi :

la première lumière d'excitation comprenant une première longueur d'onde de lumière, et la seconde lumière d'excitation comprenant une seconde longueur d'onde de lumière différente de la première longueur d'onde de lumière, ou
la première fluorescence comprenant une première longueur d'onde de lumière, et la seconde fluorescence comprenant une seconde longueur d'onde de lumière différente de la première longueur d'onde de lumière.

2. Procédé selon la revendication 1, l'orientation d'une première lumière d'excitation vers l'échantillon comprenant l'orientation d'une première lumière polarisée vers l'échantillon, et/ou la mesure du niveau d'anisotropie de la première fluorescence comprenant :

la détection d'une première fluorescence ayant une première polarisation ;
la détection d'une première fluorescence ayant une seconde polarisation qui est différente de la première polarisation ; et
la détermination du niveau d'anisotropie de la première fluorescence sur la base de la première fluorescence détectée ayant la première polarisation et de la première fluorescence détectée ayant la seconde polarisation.

3. Procédé selon la revendication 1, la détermination de la présence ou de l'absence de la première molécule d'acide nucléique cible dans l'échantillon comprenant :

la détermination que le niveau mesuré d'anisotropie de la première fluorescence est supérieur ou égal à une première valeur seuil ; et
en réponse à la détermination que le niveau mesuré d'anisotropie de la première fluorescence est supérieur ou égal à la première valeur seuil, la détermination que la première molécule d'acide nucléique cible est présente dans l'échantillon, et/ou la détermination de la présence ou de l'absence de la première molécule d'acide nucléique cible dans l'échantillon comprenant :

la détermination que le niveau mesuré d'anisotropie de la première fluorescence est inférieur à une première valeur seuil, et
en réponse à la détermination que le niveau mesuré d'anisotropie de la première fluorescence est inférieur à la première valeur seuil, la détermination que la première molécule d'acide nucléique cible est absente de l'échantillon.

4. Procédé selon la revendication 1, au moins une de la première molécule d'acide nucléique cibles ou de la seconde molécule d'acide nucléique cible étant une molécule d'acide ribonucléique (ARN).

5. Procédé selon la revendication 1, comprenant en outre la détermination d'une présence de bactéries dans l'échantillon ou d'une indication de cancer sur la base du niveau mesuré d'anisotropie de la première fluorescence, et/ou comprenant en outre la distinction entre deux ou plus de deux types de bactéries dans l'échantillon sur la base du niveau mesuré d'anisotropie de la première fluorescence.

6. Système pour détecter une première molécule d'acide nucléique cible et une seconde molécule d'acide nucléique cible dans un échantillon, le système comprenant :

un ou plusieurs processeurs informatiques ; et
un ou plusieurs ensembles de mesure couplés de manière communicative au ou aux processeurs informatiques, chaque ensemble de mesure comprenant :

une région de détection configurée pour accepter un échantillon ;
un ensemble de lumière configuré et commandé par le ou les processeurs informatiques pour orienter une première lumière d'excitation et une seconde lumière d'excitation dans l'échantillon dans la région de détection ;
un premier ensemble de photodétecteurs ; et
un second ensemble de photodétecteurs, les premier et second ensembles de photodétecteurs étant configurés et commandés par le ou les processeurs informatiques pour mesurer un niveau d'anisotropie de la première fluorescence et un niveau d'anisotropie de la seconde fluorescence émis par l'échantillon dans la région de détection, et
le ou les processeurs informatiques étant configurés pour déterminer une présence ou une absence de la première molécule d'acide nucléique cible et de la seconde molécule d'acide nucléique cible dans l'échantillon sur la base du niveau d'anisotropie de la première fluorescence et du niveau d'anisotropie de la seconde fluorescence, et

le système étant configuré pour réaliser le procédé selon l'une quelconque des revendications 1 à 5.

**7.** Système selon la revendication 6, l'ensemble de lumière comprenant une source de lumière, et un premier polariseur disposé entre la source de lumière et la région de détection, éventuellement le premier ensemble photodétecteur comprenant une première photodiode, et un deuxième polariseur disposé entre la première photodiode et la région de détection, éventuellement le second ensemble photodétecteur comprenant une seconde photodiode, et un troisième polariseur disposé entre la seconde photodiode et la région de détection,

**8.** Système selon la revendication 6, le système comprenant une pluralité d'ensembles de mesure, comprenant en outre éventuellement un ensemble de chauffage, l'ensemble de chauffage étant configuré, pendant le fonctionnement du système, pour appliquer de la chaleur à la zone de détection de chacun du ou des ensembles de mesure.

**9.** Système selon la revendication 6, comprenant en outre un émetteur-récepteur sans fil configuré pour transmettre des données et/ou recevoir des données d'un dispositif informatique externe au système, le dispositif informatique étant éventuellement un smartphone ou une tablette informatique.

**10.** Système selon la revendication 6, l'ensemble de lumière étant configuré et commandé par le ou les processeurs informatiques pour :

orienter la première lumière d'excitation ayant une première longueur d'onde dans l'échantillon dans la région de détection ; et
orienter la seconde lumière d'excitation ayant une seconde longueur d'onde dans l'échantillon dans la région de détection, la première longueur d'onde étant différente de la seconde longueur d'onde, l'ensemble de lumière étant configuré et commandé par le ou les processeurs informatiques pour orienter alternativement la première lumière d'excitation ayant la première longueur d'onde dans l'échantillon dans la région de détection et orienter la seconde lumière d'excitation ayant la seconde longueur d'onde dans l'échantillon dans la région de détection au cours d'une période de temps,
éventuellement, le ou les processeurs informatiques étant enfermés dans un boîtier, et le ou les ensembles de mesure pouvant être fixés de manière réversible au boîtier par l'intermédiaire d'une interface de communication.

**FIG. 1A**

104

Target Sequence

ACCTCGTACACCAAATTAAGCTNCGTTGCGCTTCTTGGAATGG-5'

106

112

Capture Key

```
    A T G  TAAC
  C
    A T T  ATTG TGGAGCATGTGGTTTAATTCGA-3'
```

110

118

Reporter

Primer                          120          FAM-DNA

5'-TCCTCAGAAGTTT  ------>      ☆ -TATCGTCGAGTAGATTCATG-3'
3'-AGGAGTCTTCAAATACTGTCAACTCGTTATAGCAGCTCATCTAAGTAC-5'

108                    116                    Template

**FIG. 1B**

**FIG. 2**

**FIG. 3**

**FIG. 4A**

**FIG. 4B**

EP 3 432 785 B1

**FIG. 4C**

EP 3 432 785 B1

Display

Heating Block

506

Optical Detectors

Controller **FIG. 5A**

250

308

Detector I

212

Lens
Polarizer
Filter
PD

Detector II

212

Light

Lens
Polarizer
Filter
LED

208 **FIG. 5B**

EP 3 432 785 B1

FIG. 6A

Agent Preloading     Sample Loading     Thermocycling     Mix

**FIG. 6B**

EP 3 432 785 B1

**FIG. 7A**

**FIG. 7B**

EP 3 432 785 B1

**FIG. 7C**

**FIG. 7D**

800

810

Polarization
Anisotropy
Diagnostics

PAD 100% 4:20PM

**FIG. 8A**

800

810

Patient_001

Anisortropy

77    86

14    7

100
90
80
70
60
50
40
30
20
10
0
Ch1 Ch2 Ch3 Ch4

PAD %SB 100% 4:20PM

**FIG. 8B**

800

810

Search Google Maps

Thoreau Path
Thoreau Path
Blossom St
Parkman St
Lederman Park
Massachusetts
General Hospitals
Charles/MGH
Google
25,03091,121,50437

PAD %SB 100% 4:20PM

**FIG. 8C**

900

Incubate a sample with a
reaction solution
902

Direct excitation light to the
sample
904

Measure a level of anisotropy of
the fluorescence
906

Determine a presence or an
absence of a target nucleic acid
molecule based on the level of
anisotropy of the fluorescence
908

**FIG. 9**

**FIG. 10**

EP 3 432 785 B1

**FIG. 11A**

**FIG. 11B**

**FIG. 11C**

**FIG. 14**

**FIG. 12**

**FIG. 13**

**FIG. 15**

**FIG. 16**

FIG. 19

FIG. 20

FIG. 17

FIG. 18

**HAI keys**

*Escherichia*
*Klebsiella*
*Acinetobacter*
*Pseudomnas*
*Staphylococcus*

$\Delta r\,(\times10^{-3})$

0    20    40

**FIG. 23**

| | E. coli | K. pneumoniae | A. baumannii | P. aeruginosa | S. aureus |
|---|---|---|---|---|---|
| | 35.5 | 2 | 0 | 0.7 | 0 |
| | 0.9 | 36.7 | 0.2 | 0.2 | 0.2 |
| | 1.3 | 0.9 | 36 | 1.6 | 1.3 |
| | 0.7 | 0.7 | 1.3 | 36 | 0.9 |
| | 0.9 | 0.4 | 0.4 | 1.3 | 33.3 |

---

*Escherichia* key

A T T A T T G  TGGGAGGAGGGAGTAAAGTTAAT–3′
C A T G TAAC

ACCTCCTTCCCTCATTTCAATTATG G A
A
5′—TCTGCGGGTAACGTCAATGAGC A

**Target sequence (E. coli)**

**FIG. 21**

---

*Escherichia* key

$\Delta r\,(\times10^{-3})$

40
30
20
10
0

E. coli
K. pneumoniae
A. baumannii
P. aeruginosa
S. aureus

**FIG. 22**

FIG. 24

**FIG. 25A**

**FIG. 25B**

RNA extraction with fluidic cartridge

↓

Reverse transcription
(random priming)

↓

cDNA aliquoted into ten tubes

• Bacterial burden (1 tube - UNI)
• Species typing (5 tubes - HAI)
• Resistance and virulence (4 tubes - ARV)

40 min

↓

Asymmetric PCR

• Reagents (Table S1) added to each tube
  DNA polymerase,
  UDG, dCTP, dATP, dGTP and dUTP†
  Limiting primer
  Excess primer

60 min
†UDG method

↓

Incubation with detection probes

• Reagents added (Table 1 and Table S1)
  Detection key
  Common reporter

20 min

↓

Fluorescent anisotropy measurement

1 min

# FIG. 26

**FIG. 27**

EP 3 432 785 B1

FIG. 28

FIG. 29

| Cell line | Mutation status (Cells and exosomes) |
|---|---|
| Cell lines with KRAS mutations | |
| 1) ASPC1 (Homozygous mutant) | $KRAS^{G12D}$ (G -> A) |
| 2) SW480 (Homozygous mutant) | $KRAS^{G12V}$ (G -> T) |
| 3) HCT116 (Heterozygous mutant) | $KRAS^{G13D}$ (G -> A) |

| Cell line with BRAF mutations | | |
|---|---|---|
| 4) HT29 (Heterozygous mutant) | $BRAF^{V600E}$ (T -> A) | |
| Wild type control | | |
| 5) SKBR3 (Wild) | KRAS12 and 13 | |
| | BRAF600 | |

# FIG. 30

**FIG. 31**

| ASCP1 (G12D) | |
|---|---|
| Forward Primer (w.o. N-2) | ACTTGTGGTAGTTGGAGCTGA |
| Forward Primer (w. N-2) | ACTTGTGGTAGTTGGAGCCGA |
| Reverse Primer | ATGATTCTGAATTAGCTGTATCGT |
| SW480 (G12V) | |
| Forward Primer (w.o. N-2) | ACTTGTGGTAGTTGGAGCTGT |
| Forward Primer (w. N-2) | ACTTGTGGTAGTTGGAGCCGT |
| Reverse Primer | ATGATTCTGAATTAGCTGTATCGT |

EP 3 432 785 B1

FIG. 32

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PARK et al.** *CHEMISTRY: A EUROPEAN JOURNAL,* 09 November 2015, vol. 21, 16359-16363